# EUROPEAN PATENT APPLICATION

(11) **EP 2 740 477 A1**
(43) Date of publication of application: **11.06.2014**
(21) Application number: 12819172.3
(22) Date of filing: 01.08.2012
(51) Int. Cl.: A61K 31/506, A61K 31/337, A61P 35/00, A61P 43/00, C07D 401/14

(54) **METHOD FOR TREATING CANCER BY COMBINED USE OF MEDICINAL AGENTS**

(30) Priority: 02.08.2011 JP 2011169073
(71) Applicant: Astellas Pharma Inc., Tokyo 103-8411 (JP)
(72) Inventor: ARAI, Yukinori, Tokyo 103-8411 (JP)
(74) Representative: HOFFMANN EITLE
(86) International application number: PCT/JP2012/069586
(87) International publication number: WO 2013/018829

(57) **Abstract**

[Problem]

There is a demand for a therapeutic method for enhancing the efficacy of a taxane-family anti-tumor agent against various tumors.

[Means for Solution]

The inventors conducted an investigation on the method for enhancing the anti-tumor effect of taxane-family anti-tumor agents, and as a result, the inventors confirmed that a composition for cancer treatment comprising N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide (FK330) or a salt thereof as an active ingredient, enhances the anti-tumor effect of taxane-family anti-tumor agents, thus completing the invention. That is, the invention relates to a composition for cancer treatment comprising FK330 or a salt thereof as an active ingredient, which is for combined administration with one or more anti-tumor agents selected from taxane-family anti-tumor agents.

## Description

### Technical Field

The present invention relates to a medicine, particularly to a composition for cancer treatment to be used in combination with taxane-family anti-tumor agent(s), which comprises N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof as an active ingredient.

### Background Art

In chemotherapy for malignant tumors, taxane-family anti-tumor agents, particularly paclitaxel and docetaxel, are used as effective drugs for a number of carcinomas.

Taxane-family anti-tumor agents are known to inhibit division of cancer cells by inhibiting depolymerization of microtubules, which is necessary for cell division. Paclitaxel is used in the treatment of ovarian cancer, non-small cell lung cancer, breast cancer, gastric cancer and the like, and docetaxel is used in the treatment of breast cancer, non-small cell lung cancer, gastric cancer, head and neck cancer, ovarian cancer, esophageal cancer, uterine cancer and the like.

In the clinical field, for the purpose of increasing the drug efficacy or expanding the anticancer spectrum, multi-drug combination therapy which combines a taxane-family anti-tumor agent and another anti-tumor agent having a different mechanism of action is practiced. For example, using paclitaxel and carboplatin in combination (Cancer, 1996, 77:2458-63), docetaxel and cisplatin in combination (Eur. J. Surg. Oncol. 2006, 32(3), 297-302) or the like is known. Further, in the various databases provided by the U.S. Food and Drug Administration (FDA), for example, in "Drugs @ FDA" (http://www.accessdata.fda.gov/scripts/cder/drugsatfda/index.cfm) or in the website of the European Medicines Agency (EMA) (http://www.ema.europa.eu/), clinical regimens for using paclitaxel or docetaxel with other anti-tumor agents in combination are disclosed.

In general, if the dosage of an anti-tumor agent is increased, side effects also appear proportionally thereto, and thus, it is necessary to take caution in the administration of the anti-tumor agent. Therefore, in regard to the administration of an anti-tumor agent, the maximum tolerated dose (MTD) is set from the viewpoint of side effects, and the dosage is limited. Furthermore, for example, according to the package insert of Taxol Injection (registered trademark; trade name of paclitaxel) on a website for providing information on pharmaceutical products and medical instruments in Japan, it is described that paclitaxel is "administered by intravenous infusion in an amount of 210 mg/m² once a day over 3 hours, and is withdrawn for at least 3 weeks. Administration is repeated by defining this as one cycle. Meanwhile, the dosage is appropriately increased or decreased depending on the age and symptoms." As such, paclitaxel is specified with regard to the dose as well as the administration method. Furthermore, it is also described for Taxotere Injection (registered trademark: trade name of docetaxel) that "the maximum single dose is 70 mg/m², and the dose is indicated.

On the other hand, a peptide derivative having a good nitric oxide (NO) synthase (NOS) inhibitory activity is disclosed in WO 02/055541 (Patent Document 1). Particularly, it is reported that N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide (also called FR260330 or FK330) represented by the following formula, which is disclosed in Example 41 of the relevant pamphlet, exhibits an excellent inducible nitric oxide synthase (inducible NOS, iNOS) inhibitory activity (Non-Patent Document 1). Further, it is reported that FK330 is effective as a chronic rejection model for rat aorta transplantation (Non-Patent Document 2). Also, it has already been reported that the compound is effective for monkey renal ischemia/reperfusion injury and ischemia/reperfusion injury in rat liver transplantation (Non-Patent Documents 3 and 4); that the compound suppresses fibrosis induced by an increase in TGFβ1 or activation of stellate cells and is effective for the treatment of cirrhosis of the liver (Patent Document 2 and Non-Patent Document 5); and that the compound is also effective for inflammatory keratosis, keratosis, or inflammatory skin diseases (Patent Document 3).

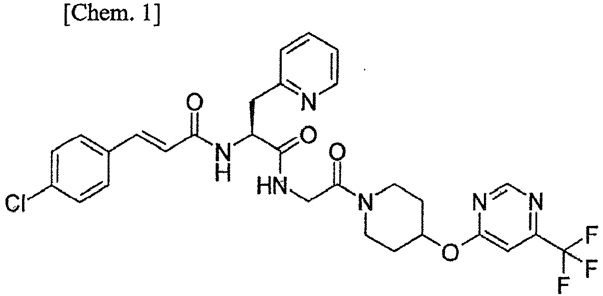

In regard to iNOS, expression is recognized in various types of cells such as epithelial cells, smooth muscle cells, macrophages and the like, and expression thereof is induced at the transcription level by cytokines or the like induced by infection, inflammation or the like, so that NO is produced. iNOS is in charge of the role as a central mediator in infections, biophylaxis, and the like.

It has been found by numerous studies conducted thus far, that NO promotes progress or metastasis of cancer, and also suppresses the progress or metastasis of cancer. It has been reported that NO's action on cancer is dependent on the activity or localization of NOS isoforms, concentration or time period of the exposure to NO, and the cellular sensitivity to NO (Non-Patent Document 6), and physiological research on cancer is being conducted in the aspects of both the promotion of NO production and the suppression of NO production. Therefore, consistent opinions have not been obtained on which of the promotion of NO production and the inhibition of NO production is better for application in cancer treatment (Non-Patent Documents 7 and 8).

Non-Patent Documents 9 and 10 describe that in human ovarian cancer cells or head and neck cancer cells, a low concentration of NO tends to suppress apoptosis caused by cisplatin and paclitaxel, while a high concentration of NO promotes apoptosis. In these documents, it is also described that combined use of an iNOS inhibitor, 1400W, and survivin RNAi promotes apoptosis caused by cisplatin as well as paclitaxel. Thus, it can be said that the cell protecting effect of NO is not a paclitaxel-selective action.

### Related Art

### Patent Document

[Patent Document 1] WO 02/055541
[Patent Document 2] Japanese Patent No. 3700854
[Patent Document 3] JP-A-2006-008621

### Non-Patent Document

[Non-Patent Document 1] European Journal of Pharmacology 509, 71, (2005)
[Non-Patent Document 2] Transplantation 79, 10 1386 (2005)
[Non-Patent Document 3] Transplantation 81, 4, 627 (2006)
[Non-Patent Document 4] American Journal of Transplantation 6, 9, 2013 (2006)
[Non-Patent Document 5] Wound Repair and Regeneration 15, 881 (2007)
[Non-Patent Document 6] Nature Reviews Cancer, 6(7), 521-34 (2006)
[Non-Patent Document 7] Cancer Chemotherapy and Pharmacology, 67, 1211-1224 (2011)
[Non-Patent Document 8] Nitric Oxide 19, 158-163 (2008)
[Non-Patent Document 9] Cancer Research, 68, 5158-5166 (2008)
[Non-Patent Document 10] International Journal of Cancer, 124,2033-2041 (2009)

### Disclosure of Invention

### Problems to Be Solved by the Invention

Taxane-family anti-tumor agents are useful for the treatment of various tumors, but due to their side effects, there are limitations on the dosage or usage. Thus, there is a strong demand for a therapeutic method which further increases the efficacy against various tumors without increasing the dosage. Also, there is a problem of acquired resistance by which the anti-tumor effect is attenuated as the treatment is continued, though the anti-tumor agent is effective at the beginning of the treatment.

### Means for Solving the Problems

The inventors of the present invention conducted a thorough investigation of the method for enhancing the anti-tumor effect of taxane-family anti-tumor agents, and as a result, they surprisingly found that when N²-[(2E)-3-(4-chlorophenyl)-2-propenoyl]-N-[2-oxo-2-(4-{[6-(trifluoromethyl)pyrimidin-4-yl]oxy}piperidin-1-ylethyl]-3-pyridin-2-yl-L-alaninamide is used in combination, significant anti-tumor effect enhancement is achieved, and found the mechanism of the enhancing effect. Thus, the inventors completed the present invention.

That is, the present invention relates to:
(1) a composition for cancer treatment, comprising FK330 or a salt thereof as an active ingredient, which is used for combined administration in combination with one or more anti-tumor agents selected from taxane-family anti-tumor agents.

Further embodiments are shown below.
(2) The composition described in item (1), which is used for combined administration in combination with paclitaxel or docetaxel.
(3) The composition described in item (2), which is used for combined administration in combination with paclitaxel.
(4) The composition described in item (2), which is used for combined administration in combination with docetaxel.
(5) The composition described in items (1) to (4), which is a composition for oral administration comprising FK330 or a salt thereof in an amount of 60 mg/day to 1200 mg/day.
(6) The composition described in item (5), which is a composition for oral administration comprising FK330 or a salt thereof in an amount of 60 mg/day to 200 mg/day.
(7) The composition described in items (1) to (6), which is used for combined administration in further combination with one or more anti-tumor agents selected from platinum-family anti-tumor agents, anthracycline-family anti-tumor agents, cyclophosphamide, fluorouracil, trastuzumab, and capecitabine.
(8) The composition described in items (1) to (6), which is used for combined administration of one or more anti-tumor agents selected from taxane-family anti-tumor agents in at least one day during one administration cycle.
(9) The composition described in item (8), which is used for combined administration of one or more anti-tumor agents selected from taxane-family anti-tumor agents in at least one of the days of administration.
(10) The composition described in item (8), which is used for combined administration of one or more anti-tumor agents selected from taxane-family anti-tumor agents for all days during one administration cycle.

Further, the present invention relates to the following inventions.
(11) The composition for cancer treatment described in items (1) to (6), which is administered with one or more anti-tumor agents selected from taxane-family anti-tumor agents simultaneously, separately, continuously, or with an interval.
(12) A composition for cancer treatment for treating a subject that is undergoing therapy with one or more anti-tumor agents selected from taxane-family anti-tumor agents, wherein the composition comprises FK330 or a salt thereof as an active ingredient.
(13) The composition for cancer treatment described in item (12), which is for the treatment of cancer in which taxane-family anti-tumor agents are used.
(14) The composition for cancer treatment described in item (13), wherein the cancer for which the taxane-family anti-tumor agents are used is non-small cell lung cancer.
(15) The composition for cancer treatment described in item (13), wherein the cancer for which the taxane-family anti-tumor agents are used is breast cancer.

Further, the present invention relates to the following inventions.
(16) A method for treating cancer, comprising combining one or more anti-tumor agents selected from taxane-family anti-tumor agents in a therapeutically effective amount that is used for combination therapy, and FK330 or a salt thereof in a therapeutically effective amount that is used for combination therapy; and administering the combination to a subject.
(17) A method for treating cancer, comprising administering one or more anti-tumor agents selected from taxane-family anti-tumor agents in a therapeutically effective amount that is used for combination therapy, and FK330 or a salt thereof in a therapeutically effective amount that is used for combination therapy, to a subject simultaneously, separately, continuously, or with an interval.

Meanwhile, the term "subject" is a human being or another animal in need of prevention or treatment thereof, and according to a certain embodiment, a human being in need of prevention or treatment thereof.

Furthermore, the present invention relates to the following inventions.
(18) Use of FK330 or a salt thereof for the manufacture of a composition for cancer treatment, which is used for combined administration in combination with one or more anti-tumor agents selected from taxane-family anti-tumor agents.
(19) FK330 or a salt thereof, for use of cancer treatment, which is used for combined administration in combination with one or more anti-tumor agents selected from taxane-family anti-tumor agents.
(20) A cancer treating agent comprising FK330 or a salt thereof as an active ingredient, which is administered in combination with one or more anti-tumor agents selected from taxane-family anti-tumor agents.
(21) An anti-tumor effect enhancer for one or more anti-tumor agents selected from taxane-family anti-tumor agents, which comprises FK330 or a salt thereof as an active ingredient.
(22) An agent for suppressing or preventing resistance for one or more anti-tumor agents selected from taxane-family anti-tumor agents, which comprises FK330 or a salt thereof as an active ingredient.
(23) A sensitivity enhancer for one or more anti-tumor agents selected from taxane-family anti-tumor agents, which comprises FK330 or a salt thereof as an active ingredient.

### Effects of the Invention

Since an enhancement of the cancer treating effect of taxane-family anti-tumor agents is achieved by combined use of the composition for cancer treatment of the present invention comprising FK330 or a salt thereof as an active ingredient and a taxane-family anti-tumor agent, the composition for cancer treatment of the present invention can be used in combination with one or more anti-tumor agents selected from taxane-family anti-tumor agents, for the treatment of various cancers to which existing taxane-family anti-tumor agents are known to be adapted. Cancers to be adapted to include solid tumors and lymphoma for which taxane-family anti-tumor agents are used.

### Brief Description of the Drawings

FIG. 1 is a graph illustrating the anti-tumor effect in single administration or combined administration of FK330 and doxorubicin in Example 1. Dox represents doxorubicin.
FIG. 2 is a graph illustrating the anti-tumor effect in single administration or combined administration of FK330 and gemcitabine in Example 1. Gem represents gemcitabine.
FIG. 3 is a graph illustrating the anti-tumor effect in single administration or combined administration of FK330 and irinotecan in Example 1. Iri represents irinotecan.
FIG. 4 is a graph illustrating the anti-tumor effect in single administration or combined administration of FK330 and carboplatin in Example 1. CBDCA represents carboplatin.
FIG. 5 is a graph illustrating the anti-tumor effect in single administration or combined administration of FK330 and paclitaxel in Example 1. PTX represents paclitaxel.
FIG. 6 is a graph illustrating the anti-tumor effect in single administration or combined administration of FK330 and docetaxel in Example 1. Doce represents docetaxel.
FIG 7 is a graph illustrating the results of Example 2 in which the anti-tumor effect enhancing action of FK330 at the time of multicycle administration of paclitaxel in human non-small cell lung cancer (Calu-6) tumor-bearing mice was investigated. PTX represents paclitaxel.
FIG. 8 is a graph illustrating the results of Example 3 in which the dose dependency of FK330 in the enhancing action on anti-tumor effect of paclitaxel was investigated in human non-small cell lung cancer (Calu-6) tumor-bearing mice. PTX represents paclitaxel.
FIG. 9 is a graph illustrating the results of Example 4 in which the administration timings of FK330 was investigated in human non-small cell lung cancer (Calu-6) tumor-bearing mice. PTX represents paclitaxel.
FIG. 10 is a graph illustrating the results of Example 5 in which the enhancing action of FK330 on anti-tumor effect of paclitaxel was investigated in human gastric cancer (AZ-521) tumor-bearing mice. PTX represents paclitaxel.
FIG. 11 is a graph illustrating the results of Example 5 in which the enhancing action of FK330 on anti-tumor effect of paclitaxel was investigated in human gastric cancer (MKN-28) tumor-bearing mice. PTX represents paclitaxel.
FIG. 12 is a graph illustrating the results of Example 5 in which the enhancing action of FK330 on anti-tumor effect of paclitaxel was investigated in human gastric cancer (NCI-N87) tumor-bearing mice. PTX represents paclitaxel.
FIG. 13 is a graph illustrating the results of Example 6 in which the enhancing action of FK330 on anti-tumor effect of paclitaxel was investigated in human breast cancer (MDA-MB-231) tumor-bearing mice. PTX represents paclitaxel.
FIG. 14 is a graph illustrating the results of Example 7 in which the inhibitory action of SNAP, which is a NO donor, on the taxane-induced tabulin polymerization action was investigated. (A) and (B) indicate a representative example of the inhibitory action of SNAP on paclitaxel-induced tubulin polymerization, and the summary values at 60 minutes after the initiation of tubulin polymerization, respectively. PTX represents paclitaxel, and SNAP represents S-nitroso-N-acetyl-D,L-penicillamine.
FIG. 15 is a graph illustrating the results of Example 7 in which the inhibitory action of SNAP, which is a NO donor, on the taxane-induced tubulin polymerization action was investigated. (A) and (B) indicate a representative example of the inhibitory action of SNAP on docetaxel-induced tubulin polymerization, and the summary values at 60 minutes after the initiation of tubulin polymerization, respectively. PTX represents docetaxel, and SNAP represents S-nitroso-N-acetyl-D,L-penicillamine.
FIG 16 is a graph illustrating the results of Example 8 in which the action of paclitaxel alone and of paclitaxel in combination with FK330 on the iNOS protein expression in the tumor tissues in human lung cancer cell line Calu-6 tumor bearing mice was investigated. PTX represents paclitaxel.
FIG. 17 is a graph illustrating the results of Example 8 in which the action of paclitaxel alone and of paclitaxel in combination with FK330 on the macrophage (F4/80) infiltration in the tumor tissues in human lung cancer cell line Calu-6 tumor-bearing mice was investigated. PTX represents paclitaxel.
FIG. 18 is a graph illustrating the results of Example 8 in which the action of paclitaxel alone and of paclitaxel in combination with FK330 on the nitrotyrosine (major end-product of NO) production in the tumor tissues in human lung cancer cell line Calu-6 tumor-bearing mice was investigated. PTX represents paclitaxel.

### Embodiments for Carrying Out the Invention

Hereinafter, the present invention will be described in detail.

The "composition for cancer treatment" of the present invention is a pharmaceutical composition for combined use with a taxane-family anti-tumor agent, and according to another embodiment, the composition of the invention is a pharmaceutical composition for enhancing the anti-tumor effect of a taxane-family anti-tumor agent.

The "cancer" in regard to the "composition for cancer treatment" of the present invention includes cancers for which taxane-family anti-tumor agents are used, that is, all solid tumors and lymphomas for which taxane-family anti-tumor agents are used. According to an embodiment, examples of the cancer include breast cancer, uterine cancer, ovarian cancer, prostatic cancer, lung cancer, gastric (gastric gland) cancer, non-small cell lung cancer, pancreatic cancer, head and neck squamous cell cancer, esophageal cancer, urinary bladder cancer, melanoma, colon cancer, renal cell cancer, non-Hodgkin's lymphoma, urothelial cancer and the like. According to another embodiment, examples of the cancer include breast cancer, ovarian cancer, prostatic cancer, gastric (gastric gland) cancer, non-small cell lung cancer and the like. According to another embodiment, examples of the cancer include breast cancer, gastric (gastric gland) cancer, non-small cell lung cancer and the like, and according to still another embodiment, examples of the cancer include breast cancer, while according to still another embodiment, examples of the cancer include non-small cell lung cancer.

According to another embodiment of breast cancer, examples thereof include HER2 positive metastatic breast cancer, HER2 negative metastatic breast cancer, HER2 positive breast cancer, and metastatic breast cancer. According to another embodiment, examples of breast cancer include HER2 negative metastatic breast cancer, and according to still another embodiment, examples thereof include HER2 positive metastatic breast cancer.

According to another embodiment of non-small cell lung cancer, examples thereof include locally advanced non-small cell lung cancer and metastatic non-small cell lung cancer. According to another embodiment, examples of non-small cell lung cancer include non-squamous cell cancer, and according to still another embodiment, examples thereof include non-squamous cell cancer in metastatic non-small cell lung cancer, while according to still another embodiment, examples thereof include squamous cell cancer in metastatic non-small cell lung cancer.

According to another embodiment of gastric cancer, examples thereof include metastatic gastric cancer, metastatic gastroesophageal junction cancer and the like.

According to another embodiment of prostatic cancer, examples thereof include metastatic castration-resistant prostatic cancer, hormone-resistant prostatic cancer and the like. According to another embodiment, examples of prostatic cancer include hormone-resistant prostatic cancer, and according to still another embodiment, examples thereof include metastatic castration-resistant prostatic cancer.

FK330 or a salt thereof as used in the present invention is a compound of Example 41 of WO 02/055541, and the compound can be easily obtained by the production method disclosed in the relevant patent document.

FK330 is an (S)-form optical isomer based on the presence of an asymmetric carbon atom, but may also include an (R)-form optical isomer.

Furthermore, a salt of FK330 according to the present invention is a pharmaceutically acceptable salt of FK330, and specific examples thereof include acid-addition salts of inorganic acids such as hydrochloric acid, hydrobromic acid, hydroiodic acid, sulfuric acid, nitric acid, phosphoric acid and the like; and organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, glutamic acid and the like. These acid-addition salts can be produced by subjecting the compound to a salt-forming reaction according to a conventional method.

Furthermore, FK330 or a salt thereof according to the present invention can be used as various hydrates or solvates, and crystal polymorphic substances. Furthermore, FK330 or a salt thereof also includes compounds labeled with various radioactive or nonradioactive isotopes.

FK330 or a salt thereof as used in the present invention is a free-form anhydride according to an embodiment. According to another embodiment, FK330 or a salt thereof is a free-form dihydrate. For example, when the free-form FK330 obtainable by the method described in Example 41 of WO 02/055541 is subjected to recrystallization in an alcohol/water solvent system, for example, in ethanol/water, isopropanol/water or the like, and the crystals thus obtained are dried under reduced pressure, a free-form anhydride may be obtained. Furthermore, crystals of a free-form dihydrate can be obtained by subjecting the free-form anhydride thus obtained to a humidifying operation.

A pharmaceutical composition comprising FK330 or a salt thereof as an active ingredient can be prepared by using excipients, that is, pharmaceutical excipients and pharmaceutical carriers, which are conventionally used in the art, according to a conventionally used method.

Administration may be achieved by oral administration by means of tablets, pills, capsules, granules, powders, liquids, and the like; or by parenteral administration by means of intraarticular, intravenous, and intramuscular injectable preparations, and the like. According to an embodiment, administration is achieved by oral administration by means of tablets, pills, capsules, granules, powders, liquids, and the like.

As solid compositions for oral administration, tablets, powders, granules, and the like are used. In such a solid composition, one kind or two or more kinds of active ingredients are mixed with at least one inert excipient. The composition may further contain inert additives, for example, a lubricating agent, a disintegrant, a stabilizer, and a solubilizing agent, according to a conventional method. Tablets or pills may be coated with a sugar coating, or a film of a gastric or enteric substance, as necessary.

Liquid compositions for oral administration include pharmaceutically acceptable emulsions, solutions, suspensions, syrups, elixirs and the like, and such a liquid composition contains an inert diluent which is generally used, for example, purified water or ethanol. The liquid composition may further contain adjuvants such as a solubilizer, a wetting agent, or a suspending agent, a sweetening agent, a flavoring agent, an aromatic agent, and an antiseptic agent, in addition to the inert diluents.

Injectable preparations for parenteral administration include sterile aqueous or non-aqueous solutions, suspensions or emulsions. Examples of aqueous solvents include distilled water for injection, and physiological saline. Examples of non-aqueous solvents include alcohols such as ethanol. Such a composition may further contain an isotonizing agent, an antiseptic agent, a wetting agent, an emulsifier, a dispersant, a stabilizer, or a solubilizing agent. These preparations are sterilized by, for example, filtration through a bacteria-retaining filter, incorporation of a disinfectant, or irradiation. Furthermore, these preparations can be prepared by preparing a sterile solid composition, and dissolving or suspending the solid composition in sterile water or a sterile solvent for injection before use.

The content may vary depending on the route of administration, formulation, site of administration, and the type of excipients or additives, but the pharmaceutical composition of the present invention contains one kind or more kinds of FK330 or salts thereof as active ingredients, in an amount of 0.01 wt% to 100 wt%, and according to an embodiment, in an amount of 0.01 wt% to 50 wt%.

Usually, in the case of oral administration, the daily dose of FK330 (dihydrate) is about 60 mg/day to 1200 mg/day, and this is administered once or in two to three divided portions. According to another embodiment, the daily dose is 60 mg to 200 mg, and according to still another embodiment, the daily dose is 120 mg to 250 mg, 120 to 220 mg, 150 mg to 250 mg, or 180 mg to 200 mg. According to another embodiment, the daily dose is 100 mg to 180 mg, or 180 mg to 300 mg. According to still another embodiment, the daily dose is 120 mg, 130 mg, 140 mg, 150 mg, 160 mg, 170 mg, 180 mg, 190 mg, 200 mg, 210 mg, 220 mg, 230 mg, 240 mg, or 250 mg. According to still another embodiment, the daily dose is 180 mg, 190 mg, or 200 mg. The dosage is appropriately determined according to individual cases, by taking into consideration the symptoms, age, gender, and the like.

The taxane-family anti-tumor agent that can be used in combination with FK330 of the present invention may be a pharmaceutical composition comprising a compound having a taxane ring or a structure related thereto, as an active ingredient. According to another embodiment, the taxane-family anti-tumor agent may be a pharmaceutical composition comprising a compound having a taxane ring as an active ingredient. Another embodiment of the taxane-family anti-tumor agent may be a pharmaceutical composition comprising a compound having a microtubule depolymerization inhibitory action as an active ingredient. Regarding the excipients and formulation applied to the pharmaceutical compositions, those described above can be used.

Specific examples of the taxane-family anti-tumor agent that can be used in combination with FK330 include paclitaxel, docetaxel, carbazitaxel, abraxane, tesetaxel, ortataxel and the like, and according to another embodiment, examples thereof include paclitaxel, docetaxel, and abraxane. According to still another embodiment, examples thereof include paclitaxel and abraxane, and according to still another embodiment, examples thereof include paclitaxel, while according to another embodiment, examples thereof include docetaxel.

Principal existing taxane-family anti-tumor agents that can be used in combination with FK330 of the present invention will be listed in the following table, together with principal applicable carcinomas or applicable candidate carcinomas. However, in the combination therapy of the present invention, the applicable carcinomas related to these anti-tumor agents are not limited to these.

**[Table 1]**

| Anti-tumor agent | Principal applicable (candidate) carcinoma |
|---|---|
| Paclitaxel | Ovarian cancer, non-small cell lung cancer, breast cancer, gastric (gastric gland) cancer, uterine cancer |
| Docetaxel | Breast cancer, hormone-resistant prostatic cancer, non-small cell lung cancer, gastric (gastric gland) cancer, head and neck squamous cell cancer, ovarian cancer, esophageal cancer, uterine cancer |
| Carbazitaxel | Hormone-resistant prostatic cancer |
| Abraxane (paclitaxel-albumin stabilized, small-sized particulate formulation) | Breast cancer |
| Tesetaxel (in development) | Breast cancer, gastric cancer, urinary bladder cancer, prostatic cancer, melanoma, colon cancer |
| Ortataxel (in development) | Non-small cell lung cancer, renal cell cancer, breast cancer, non-Hodgkin's lymphoma |

Regarding paclitaxel, docetaxel, carbazitaxel, or abraxane, commercially available products can be purchased and used, or can be easily obtained by a method obvious to those ordinarily skilled in the art, or a modification thereof.

Carbazitaxel can be easily obtained by the production method disclosed in WO 96/30355, or a modification thereof. Tesetaxel can be easily obtained by a method obvious to those ordinarily skilled in the art, or the production method disclosed in WO 01/27115, or a modification thereof. Ortataxel can be easily obtained by a method obvious to those ordinarily skilled in the art, or the production method disclosed in US Patent No. 5705508, or a modification thereof.

The anti-tumor agents described above have already been clinically used, and the route of administration, administration cycle, and dosage thereof are clearly known to those ordinarily skilled in the art. Suitable dosage and administration may vary with the carcinoma, symptoms, and the drug to be used in combination, and this detailed information can be easily obtained from various databases provided by the FDA, for example, "Orange Book" (http://www.fda.gov/cder/orange/default.htm), or from a website for providing information on pharmaceutical products and medical instruments in Japan (http://www.info.pmda.go.jp/). The information of various anti-tumor agents in these databases are incorporated in the present application.

For example, regarding an example of the dosage and administration of paclitaxel, according to the package insert for Taxol Injection (registered trademark; trade name of paclitaxel) from a website for providing information on pharmaceutical products and medical instruments in Japan, it is described "[Efficacy or effect] ovarian cancer, non-small cell lung cancer, breast cancer, gastric cancer; [Dosage and administration]; 1. Usually, for an adult, the agent in terms of paclitaxel is administered by intravenous infusion in an amount of 210 mg/m² once a day over 3 hours, and is withdrawn for at least 3 weeks. Administration is repeated by defining this as one cycle. Meanwhile, the dosage is appropriately decreased depending on the age and symptoms."

Furthermore, regarding an example of the dosage and administration of docetaxel, according to the package insert for Taxotere Injection (registered trademark: trade name of docetaxel) from a website for providing information on pharmaceutical products and medical instruments in Japan, it is described "1. Breast cancer, non-small cell lung cancer, gastric cancer, head and neck cancer; Dosage and administration according to efficacy or effect; 1. Usually, for an adult, the agent in terms of docetaxel is administered by intravenous infusion in an amount of 60 mg/m² once a day over 1 hour, at an interval of 3 to 4 weeks. Meanwhile, the dosage is appropriately increased or decreased depending on the symptoms. However, the maximum single dose is 70 mg/m²."

The effective therapeutic dose in the case of being used in the combination therapy according to the present invention can be usually set based on the route of administration to be administered, usually in a dosage equal to that in the case where the anti-tumor agent is administered alone, or a lower dosage than that (for example, 0.10 to 0.99 times the maximum amount of administration in the case of being administered alone). In the case of a taxane-family anti-tumor agent that is administered by a constant administration cycle, the administration cycle can be appropriately adjusted so as to be appropriate for combined use with FK330. The specific frequency of administration, dosage, administration cycle, and the like are appropriately determined in accordance with individual cases by taking into consideration the symptoms, age, gender and the like of the patient.

The form of administration in the case of combination administration of FK330 and a taxane-family anti-tumor agent is not particularly limited as long as the route of administration, frequency of administration, and dosage suitable for the respective components are employed, and examples of the form of administration include: (1) a composition comprising FK330 and a taxane-family anti-tumor agent, that is, single-agent administration; (2) simultaneous administration by the same route of administration, of two preparations obtainable by separately formulating FK330 and a taxane-family anti-tumor agent; (3) administration with a time interval by the same route of administration, of two preparations obtainable by separately formulating FK330 and a taxane-family anti-tumor agent (for example, administration in order of FK330 and the taxane-family anti-tumor agent, or administration in the reverse order); (4) simultaneous administration by different routes of administration, of two preparations obtainable by separately formulating FK330 and a taxane-family anti-tumor agent; (5) administration with a time interval by different routes of administration, of two preparations obtainable by separately formulating FK330 and a taxane-family anti-tumor agent (for example, administration in order of FK330 and the taxane-family anti-tumor agent, or administration in the reverse order) and the like. According to an embodiment, the composition for cancer treatment of the present invention is formulated separately from the taxane-family anti-tumor agent, and the taxane-family anti-tumor agent is administered through intravenous injection, intravenous infusion, or orally, while the composition for cancer treatment of the present invention is administered orally or parenterally. According to another embodiment, the taxane-family anti-tumor agent is administered by intravenous injection or intravenous infusion, while the composition for cancer treatment of the present invention is administered orally.

A preferred form of administration of the composition for cancer treatment of the present invention is a method of orally or parenterally administering the composition for cancer treatment of the present invention in at least one day during one cycle of administration of the taxane-family anti-tumor agent. Here, the term "cycle" means a treatment cycle or treatment course based on a certain regimen, and for example, in the case of paclitaxel or docetaxel, the cycle is a unit period of administration of about 3 to 4 weeks which is from the initiation of administration to the next administration, with a washout period therebetween. The form of administration according to a certain embodiment is a method of orally administering the composition for cancer treatment of the present invention comprising FK330 or a salt thereof in at least one of the administration days of the taxane-family anti-tumor agent; a method of orally administering the composition for cancer treatment of the present invention for all days of the days of administration of the taxane-family anti-tumor agent; a method of orally administering the composition for cancer treatment of the present invention in at least one day of withdrawal days of the taxane-family anti-tumor agent; a method of administering the composition for cancer treatment of the present invention for all days of the withdrawal days of the taxane-family anti-tumor agent; a method of orally administering the composition for cancer treatment of the present invention in at least one of the administration days and at least one day of the withdrawal days of the taxane-family anti-tumor agent; a method of orally administering the composition for cancer treatment of the present invention for all days of one cycle of administration of the taxane-family anti-tumor agent; or a method of orally administering the composition for cancer treatment of the present invention intermittently (once in two days, one in one week, or the like) during one cycle of administration of the taxane-family anti-tumor agent. The form of administration according to an embodiment is a method of orally administering the composition for cancer treatment according to the present invention for all days during one cycle of administration of the taxane-family anti-tumor agent. The composition for cancer treatment of the present invention may be administered several days or one day before the administration of the taxane-family anti-tumor agent, and this embodiment is included in the administration on a withdrawal day of the taxane-family anti-tumor agent. When there is a concern about drug interaction between FK330 and the taxane-family anti-tumor agent, the administration timings can be separated in order to avoid interaction, and/or administration can be carried out by leaving a necessary interval therebetween.

In the "composition for cancer treatment for treating a subject undergoing therapy with one or more anti-tumor agents selected from taxane-family anti-tumor agents, wherein the composition comprises FK330 or a salt thereof as an active ingredient" of the present invention, the "subject" may be subjected to administration of another anti-tumor agent as long as the subject is undergoing therapy with one or more anti-tumor agents selected from taxane-family anti-tumor agents. Examples of the other anti-tumor agent include the compounds which are described later. The "subject" may be administered with one or more anti-tumor agents selected from taxane-family anti-tumor agents with the composition for cancer treatment comprising FK330 or a salt thereof as an active ingredient, simultaneously, separately, continuously or with an interval.

The present invention may be in the form of a "kit including (a) FK330 or a salt thereof, and (b) one or more anti-tumor agents selected from taxane-family anti-tumor agents in combination." This includes a preparation comprising the active ingredient (a) (preparation 1) and a preparation comprising the active ingredient (b) (preparation 2) in combination so as to be used for combination therapy of these active ingredients. The kit of the present invention may be a package product which includes, as desired, additional preparations or indication members that facilitate the administration in accordance with the respective timings of administration, such as a placebo.

The two kinds of preparations are, for example, administered by the same or different routes of administration, simultaneously, separately, continuously or with an interval, under the administration conditions such as preparation formulation, route of administration, frequency of administration or the like, which are appropriate for the respective preparations, while considering the bioavailability, stability and the like of the respective preparations. Here, the term "simultaneously" means administration of the preparation 1 and the preparation 2 together by the same route of administration. The term "separately" means that the preparation 1 and the preparation 2 are administered separately by the same or different routes of administration, and at the same or different frequency of administration or interval of administration. The term "continuously" means that the preparation 1 and the preparation 2 are administered by the same or different routes of administration, in the order of the preparation 1 followed by the preparation 2, or in the reverse order. The term "with an interval" means that the preparation 1 and the preparation 2 are administered by the same or different routes of administration, in the order of the preparation 1 followed by the preparation 2 with a certain interval therebetween, or in the reverse order.

The present invention may be in the form of a "pharmaceutical product for cancer treatment, including (a) a composition comprising FK330 or a salt thereof as an active ingredient, and (c) a package insert indicating the combined use with one or more anti-tumor agent selected from taxane-family anti-tumor agents." This means a pharmaceutical product for cancer treatment which is packaged to include both the composition (a) comprising a therapeutically effective amount of the active ingredient, and the package insert (c) related to the composition of (a), which indicates combined use with one or more anti-tumor agents selected from taxane-family anti-tumor agents, in the case of using the pharmaceutical product in combination therapy.

Furthermore, when the composition for cancer treatment of the present invention comprising FK330 or a salt thereof as an active ingredient is used in combination with a taxane-family anti-tumor agent, another anti-tumor agent that is used in chemotherapy can also be used in combination if desired. The other anti-tumor agent that can be used in combination and is used in chemotherapy, is an anti-tumor agent known to be used in combination with a conventional taxane-family anti-tumor agent, and the other anti-tumor agent may be, for example, one or more anti-tumor agents selected from platinum-family anti-tumor agents, anthracycline-family anti-tumor agents, cyclophosphamide, fluorouracil, trastuzumab, and capecitabine. According to an embodiment, the other anti-tumor agent is cisplatin or carboplatin, and according to another embodiment, the other anti-tumor agent is doxorubicin.

As described in Examples 1 to 6 of the present specification, it was found that the cancer treatment effect is enhanced by combination therapy of the composition for cancer treatment of the present invention comprising FK330 or a salt thereof as an active ingredient, and a taxane-family anti-tumor agent. The effect was confirmed as the combination therapy resulted in a significant decrease in the mean tumor volume as compared with the case of single-agent administration of the composition for cancer treatment comprising FK330 or a salt thereof as an active ingredient, or the taxane-family anti-tumor agent.

Furthermore, FK330 or a salt thereof of the present invention does not have serious side effects that are reported in many anti-tumor agents, and is also useful from the viewpoint of safety.

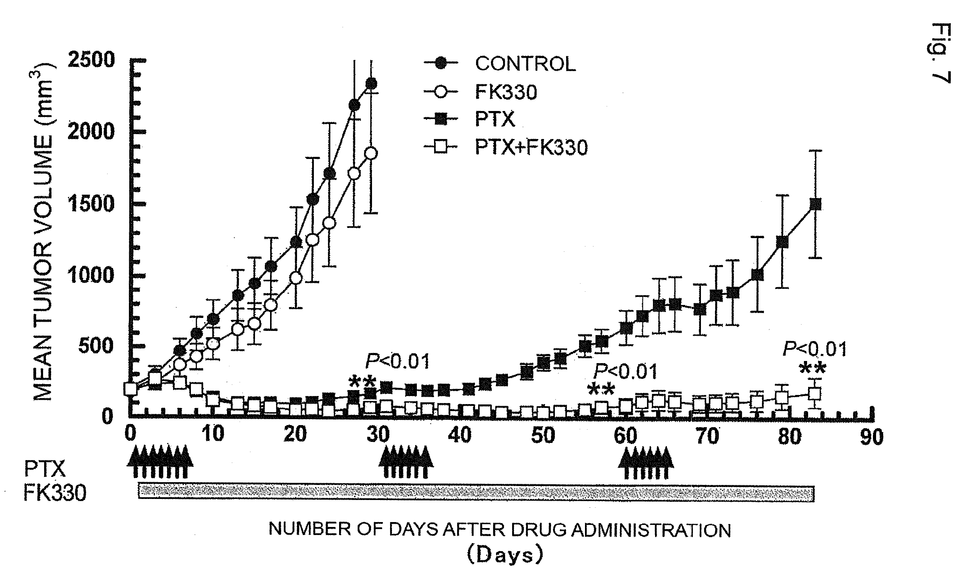

In the animal models described in Examples, it was confirmed that the composition for cancer treatment of the present invention enhances the anti-tumor effect in a taxane-family anti-tumor agent-selective and dose-dependent manner. That is, in the case of using FK330 or a salt thereof only, a significant anti-tumor effect is not observed, and any significant anti-tumor effect-enhancing action is not confirmed even if FK330 or a salt thereof is used in combination with an anti-tumor agent other than taxane. However, when FK330 or a salt thereof is combined with a taxane-family anti-tumor agent, a good anti-tumor effect-enhancing action was confirmed. Particularly, it was found that FK330 remedies the attenuation of the anti-tumor effect of taxane-family drugs caused by their multicycle treatment, and well suppresses tumor proliferation (see FIG. 7). This suggests that FK330 suppresses resistance to taxane-family anti-tumor agents.

In Examples 7 and 8 of the present specification, it is disclosed that NO directly suppresses the microtubule depolymerization inhibitory action of taxane-family anti-tumor agents, and that taxane-family anti-tumor agents induce NO production along with the expression of iNOS proteins in cancer tissues, while FK330 suppresses the iNOS activity.

Furthermore, the inventors of the present invention also found that NO suppresses the cancer cell proliferation suppressing action involving apoptosis exhibited by taxane-family anti-tumor agents, and the NO-induced attenuating effect of chemotherapeutic agents is selectively recognized in taxane-family anti-tumor agents.

Therefore, the inventors of the present invention clarified the following as the mechanism of an enhancement of the anti-tumor effect of taxane-family anti-tumor agents exhibited by FK330. That is, a taxane-family anti-tumor agent promotes iNOS protein in tumor tissues, and consequently enhances NO production. Produced NO directly suppresses the microtubule depolymerization inhibitory action of taxane, and thereby causes attenuation of the anti-tumor effect of the taxane-family anti-tumor agent. On the other hand, it has been found that FK330 inhibits NO production that has been enhanced by the taxane-family anti-tumor agent, and thereby suppresses attenuation of the effect of the taxane-family anti-tumor agent, while enhancing the anti-tumor effect.

As discussed above, it can be said that FK330 or a salt thereof is capable of enhancing the action of all taxane-family anti-tumor agents intended for the microtubule depolymerization inhibitory action.

### EXAMPLES

The pharmacological test results exhibiting usefulness of the composition for cancer treatment of the present invention are described below, but the present invention is not limited to these Examples.

### Example 1

### 1) Test substances

A free-form dihydrate of FK330 was used. Hereinafter, the amount of FK330 administered is expressed in weight of the dihydrate. A docetaxel hydrate injectable preparation (Taxotere (trademark), for intravenous infusion) was purchased from Sanofi-Aventis SA, and was used as docetaxel. A paclitaxel injectable preparation (paclitaxel injection liquid "Sawai") was purchased from Sawai Pharmaceutical Co., Ltd., and was used as paclitaxel. Irinotecan hydrochloride (Topotesin (trade name) injection) was purchased from Daiichi Sankyo Co., Ltd., and was used as irinotecan. Gemcitabine hydrochloride for injection (Gemzar (trade name) injection) was purchased from Eli Lilly Japan K.K., and was used as gemcitabine. Doxorubicin hydrochloride for injection (Adriacin (trade name) Injection 10) was purchased from Kyowa Hakko Kogyo Co., Ltd., and was used as doxorubicin. A carboplatin injection liquid (Paraplatin (trade name) injection liquid) was purchased from Bristol Myers Squibb Co., and was used as carboplatin.

### 2) Preparation and administration of test substances

FK330 was suspended (20 mg/mL) in a 0.5% aqueous methyl cellulose solution (0.5% MC). Docetaxel was prepared in the attached dissolution liquid at 4 mg/kg, and was diluted with physiological saline (Otsuka Pharmaceutical Group) to 1 mg/mL. Paclitaxel, irinotecan, gemcitabine, doxorubicin, and carboplatin were prepared with physiological saline for injection at the time of use at concentrations of 1 mg/mL, 3 mg/mL, 16 mg/mL, 1 mg/mL, and 6 mg/mL, respectively.

### 3) Cells

Human lung cancer-derived Calu 6 (HTB-56) was obtained from American Type Culture Collection (VA, USA). The cells were cultured under the conditions of 37°C and 5% CO₂ by using RPMI1640 medium containing 10% heat-inactivated fetal bovine serum (FBS). The cells were harvested with trypsin and were suspended in PBS at 6×10⁷ cells/mL, and the suspension was mixed with an equal amount of Matrigel (trade name) Basement Membrane Matrix (manufactured by Becton Dickinson Co., Bedford, MA, USA).

### 4) Animals

Five-week old male nude mice (CAnN Cg-Foxn1nu/CrlCrlj(nu/nu)) were purchased from Charles River Laboratories Japan, Inc. (Japan, Kanagawa). The animals were raised under specific pathogen-free (SPF) conditions throughout the test period by feeding standard feedstuff and drinking water. The cultured cells were subcutaneously inoculated into the dorsal region of the nude mice at a concentration of 3×10⁶ cells/0.1 mL/mouse. Nude mice which had the tumor volumes (minor axis × [major axis]² × 0.5) ranging from 111.9 mm³ to 369.3 mm³ were assigned by using SAS to minimize the fluctuation in tumor volume between the groups and within the group.

### 5) Drug administration and measurement

The first day of administration was designated as Day 1, and observation was made until Day 29. The respective groups (n = 5 or 6) were treated as follows. FK330 was orally gavaged (5 mL/kg) twice a day at an interval of 6 to 8 hours, and each of the chemotherapeutic agents was administered by intravenous bolus injection (10 mL/kg) through the tail vein.

Control group: Untreated
FK330 single administration group: 100 mg/kg of FK330 was administered twice a day (bid) (200 mg/kg/day, administered every day from the initiation of experiment to the completion)
Docetaxel single administration group: Docetaxel 10 mg/kg/day (Day 1,5 and 9)
Paclitaxel single administration group: Paclitaxel 10 mg/kg/day (Day 1 through 7)
Irinotecan single administration group: Irinotecan 30 mg/kglday (Day 1 through 7)
Gemcitabine single administration group: Gemcitabine 160 mg/kg/day (Day 1, 4 and 7)
Doxorubicin single administration group: Doxorubicin 10 mg/kg/day (Day 1 and 8)
Carboplatin single administration group: Carboplatin 60 mg/kg/day (Day 1 and 2)

Combined use group:
FK330 100 mg/kg/day bid + Docetaxel 10 mg/kg/day (Day 1, 5 and 9), n = 6
FK330 100 mg/kg/day bid + Paclitaxel 10 mg/kg/day (Day 1 through 7), n = 6
FK330 100 mg/kg/day bid + Irinotecan 30 mg/kg/day (Day 1 through 7), n = 6
FK330 100 mg/kg/day bid + Gemcitabine 160 mg/kg/day (Day 1, 4 and 7), n = 6
FK330 100 mg/kg/day bid + Doxorubicin 10 mg/kg/day (Day 1 and 8), n = 6
FK330 100 mg/kg/day bid + Carboplatin 60 mg/kg/day (Day 1 and 2), n = 5
FK330 was orally gavaged (5 mL/kg) twice a day at an interval of 6 to 8 hours.
Each of the chemotherapeutic agents was administered by intravenous bolus injection (10 mL/kg) through the tail vein 2 to 4 hours after the administration of FK330.

Every 3 to 4 days, the body weight and the tumor diameter, determined by using a digital caliper, were measured. The tumor volume was calculated from the calculation formula for ellipsoid volume of (minor axis × [major axis]² × 0.52).

### 6) Statistical analysis

The results are expressed as the mean value (Mean) ± standard error (SEM). During the test period, a value less than or equal to the palpation limit was defined as complete remission (CR), and the measurement value was designated as 0. Using the actual measured values of the tumor volume at the end of the study, an unpaired statistical test (Student's t-test) of the difference between two groups was carried out between a single administration group and a combined use group. A difference value of less than 5% was considered to be statistically significant. For the data processing, SAS or Graphpad Prism version 5.03 was used.

### 7) Results

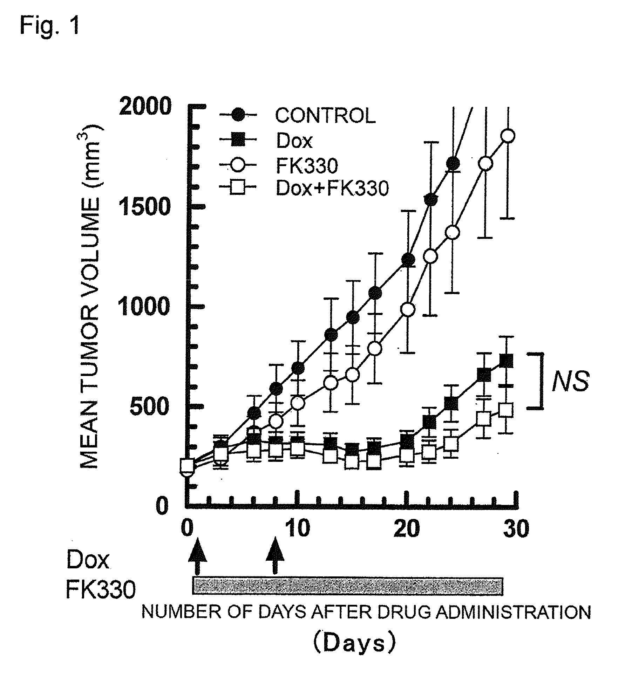

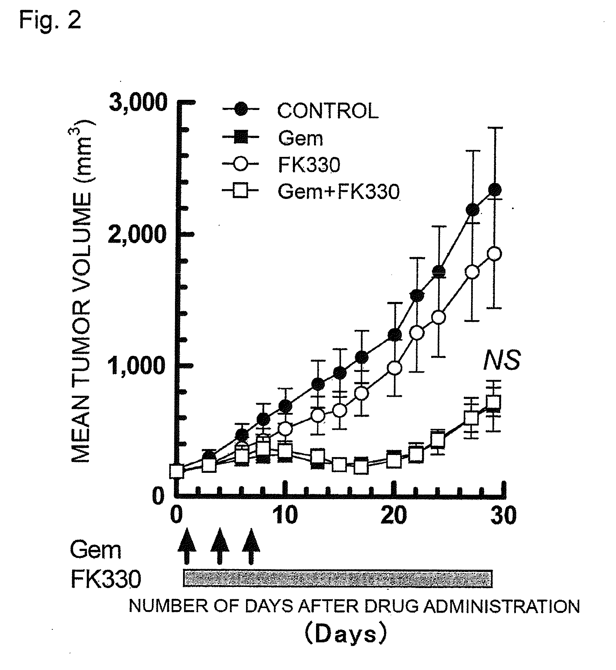

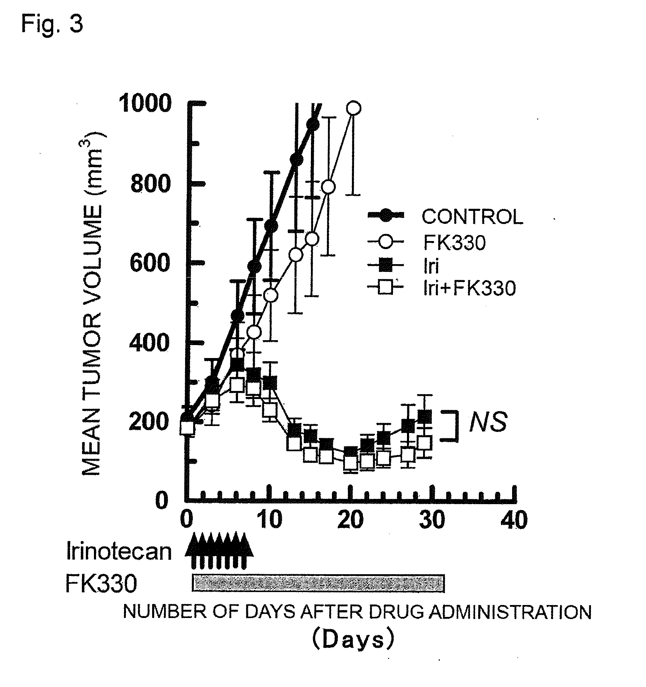

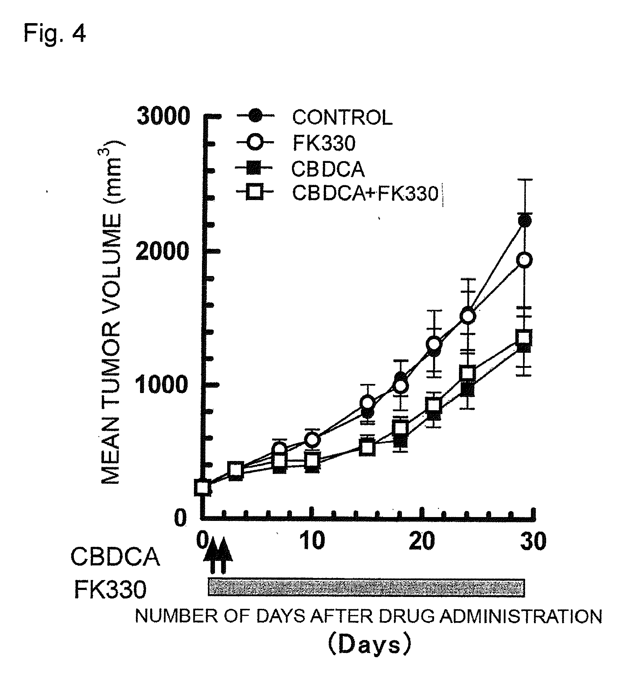

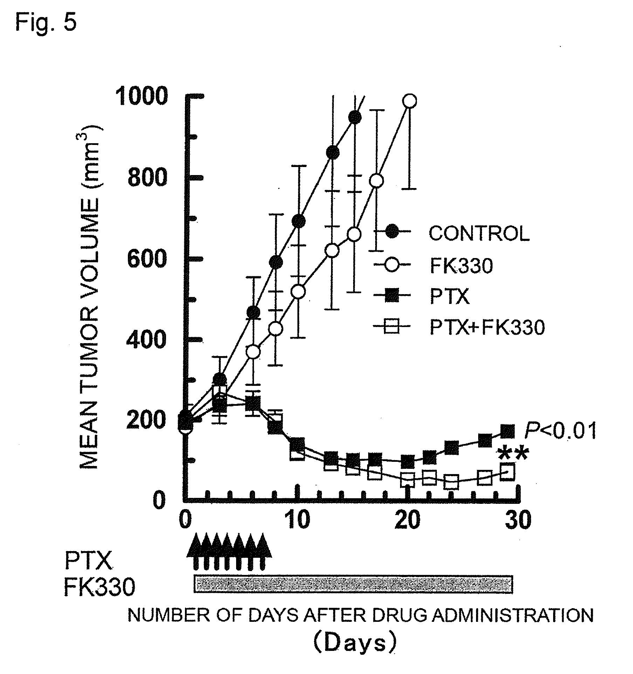

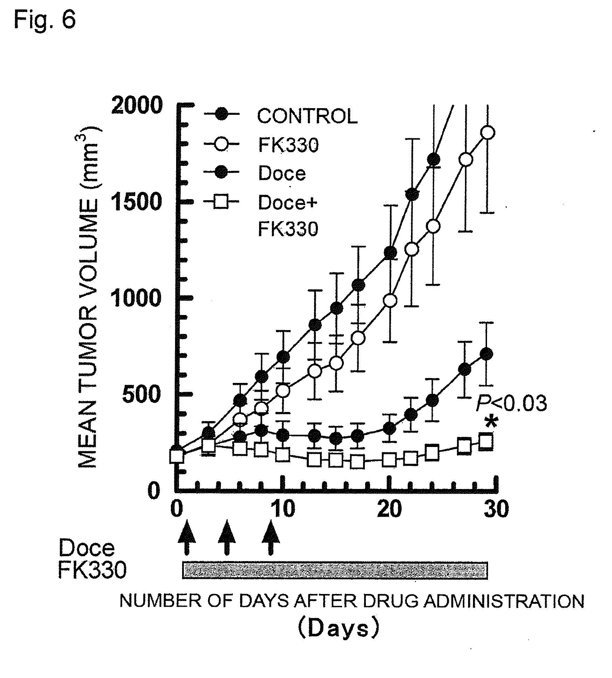

The anti-tumor effect of combined administration of FK330 and various chemotherapeutic agents was investigated. The results are shown in FIG. 1 to FIG. 6. That is, the combined use effect of FK330 and doxorubicin is shown in FIG. 1; the combined use effect with gemcitabine is shown in FIG. 2; the combined use effect with irinotecan is shown in FIG. 3; the combined use effect with carboplatin is shown in FIG. 4; the combined use effect with paclitaxel is shown in FIG. 5; and the combined use effect with docetaxel is shown in FIG. 6. An anti-tumor effect was not recognized when FK330 was used alone, but a significant enhancement of the anti-tumor effect was recognized in paclitaxel (tumor volume reduction ratio compared with paclitaxel-only group on Day 29: 57.6%) and docetaxel (tumor volume reduction ratio compared with docetaxel-only group on Day 29: 65.2%). On the other hand, FK330 did not affect the anti-tumor effect of gemcitabine and carboplatin. A slight enhancement of the anti-tumor effect was observed in doxorubicin (tumor volume reduction ratio compared with doxorubicin-only group on Day 29: 34.5%) and irinotecan (tumor volume reduction ratio compared with irinotecan-only group on Day 29: 34.1%), but the effects were not significant.

The results are expressed as the mean value (Mean) ± standard error (SEM) (n = 6). *: P < 0.03 and **: P < 0.01 represent the significant differences obtained as compared between the combined use groups and the chemotherapeutic agent single administration groups. N.S.: represents that there was no significant difference as compared between the combined use groups and the chemotherapeutic agent single administration groups (Student's t-test).

### 8) Conclusions

FK330 by itself was not found to have an anti-tumor effect, but enhanced the anti-tumor effect of paclitaxel and docetaxel. On the other hand, FK330 did not affect the anti-tumor effect of irinotecan, gemcitabine, doxorubicin and carboplatin. These results suggest that FK330 selectively enhances the anti-tumor effect of taxane-family anti-tumor agents.

### Example 2

### 1) Test substances

The dose of FK330 is expressed in weight including the hydrate. A paclitaxel injectable preparation (paclitaxel injection liquid, "Sawai") was purchased from Sawai Pharmaceutical Co., Ltd., and was used as paclitaxel.

### 2) Preparation and administration of test substances

FK330 was suspended (20 mg/mL) in a 0.5% aqueous methyl cellulose solution (0.5% MC). Paclitaxel was prepared with physiological saline for injection (Otsuka Pharmaceutical Group) to a concentration of 1 mg/mL at the time of use.

### 3) Cells

Human lung cancer-derived Calu 6 (HTB-56) was obtained from American Type Culture Collection (VA, USA). The cells were cultured under the conditions of 37°C and 5% CO₂ by using RPMI1640 medium containing 10% heat-inactivated fetal bovine serum (FBS). The cells were harvested with trypsin and were suspended in PBS at 6×10⁷ cells/mL, and the suspension was mixed with an equal amount of Matrigel (trade name) Basement Membrane Matrix (manufactured by Becton Dickinson Co., Bedford, MA, USA).

### 4) Animals

Five-week old male nude mice (CAnN Cg-Foxn1nu/CrlCrlj(nu/nu)) were purchased from Charles River Laboratories Japan, Inc. (Japan, Kanagawa). The animals were raised under specific pathogen-free (SPF) conditions throughout the test period by feeding standard feedstuff and drinking water. The cultured cells were subcutaneously inoculated into the dorsal region of the nude mice at a concentration of 3×10⁶ cells/0.1 mL/mouse. Nude mice which had the tumor volumes (minor axis × [major axis]² × 0.5) ranging from 126.1 mm³ to 299.0 mm³ were assigned by using SAS to minimize the fluctuation in the tumor volume between the groups and within the group.

### 5) Drug administration and measurement

The first day of administration was designated as Day 1, and observation was made until Day 83. The respective groups (n = 6) were treated as follows. FK330 was orally gavaged in an amount of 5 mL/kg, and paclitaxel was administered intravenously through the tail vein in an amount of 10 mL/kg. Paclitaxel was continuously administered from Day 1 to Day 7, and then was withdrawn for 24 days (1 cycle). In the administration of PTX in the second cycle and the third cycle, PTX was continuously administered for 6 days from Day 31 and from Day 60, respectively.

### Control group: Untreated

FK330 single administration group: FK330 100 mg/kg bid (200 mg/kg/day, continuously administered from the initiation of experiment to the completion of experiment)

Paclitaxel single administration group: Paclitaxel 10 mg/kg/day (continuously administered for 7 days in Cycle 1, and continuously administered for 6 days in Cycle 2 and the subsequent cycle)

Combined use group:
FK330 100 mg/kg/kg bid (200 mg/kg/day, continuously administered from the initiation of experiment to the completion of experiment) + paclitaxel 10 mg/kg/day (continuously administered for 7 days in Cycle 1, and continuously administered for 6 days in Cycle 2 and the subsequent cycle)
FK330 was orally gavaged (5 mL/kg) twice a day at an interval of 6 to 8 hours. Paclitaxel was administered by intravenous bolus injection (10 mL/kg) through the tail vein 2 to 4 hours after the administration of FK330.

Every 3 to 4 days, the body weight and the tumor diameters, determined by using a digital caliper, were measured. The tumor volume was calculated from the calculation formula for ellipsoid volume of (minor axis × [major axis]² × 0.52).

### 6) Statistical analysis

The results are expressed as the mean value (Mean) ± standard error (SEM). During the test period, a value less than or equal to the palpation limit was defined as complete remission (CR), and the measurement value was designated as 0. Using the actual measured values of the tumor volume at the end of the study, an unpaired statistical test (Student's t-test) of the difference between two groups was carried out between a single administration group and a combined use group. A difference value of less than 5% was considered to be statistically significant. For the data processing, SAS was used.

### 7) Results

The combined use action of FK330 at the time of multicycle treatment of paclitaxel was investigated. The results are shown in FIG. 7. In human non-small cell lung cancer (Calu-6) tumor-bearing mice, it was found that the anti-tumor effect of paclitaxel tended to attenuate as a result of multicycle administration. FK330 enhanced the anti-tumor effect of paclitaxel very clearly, and the tumor volume reduction ratio compared with the paclitaxel-only group on Day 29 was 57.6% (P < 0.01), the tumor volume reduction ratio compared with the paclitaxel-only group on Day 60 was 86.1% (P < 0.01), while the tumor volume reduction ratio compared with the paclitaxel-only group on Day 83 was 87.6% (P < 0.01).

The results are expressed as the mean value (Mean) ± standard error (SEM) (n = 6). **: P < 0.01 represents the significant difference obtained as compared between the combined use group and the chemotherapeutic agent single administration group (Student's t-test).

### 8) Conclusions

In human non-small cell lung cancer (Calu-6) tumor-bearing mice, the anti-tumor effect of paclitaxel attenuated in a cycle-dependent manner. On the other hand, combined use of FK330 enhanced the anti-tumor effect of paclitaxel under the multicycle administration, and very clearly suppressed regrowth of tumors. These results suggest that FK330 remedies attenuation of the anti-tumor effect of a taxane-family drug through the multicycle treatment.

### Example 3

Preparation of the test substances and conditioning of the cells were carried out in the same manner as in Example 2.

### 1) Animals

Five-week old male nude mice (CAnN Cg-Foxn1nu/CrlCrlj(nu/nu)) were purchased from Charles River Laboratories Japan, Inc. (Japan, Kanagawa). The animals were raised under specific pathogen-free (SPF) conditions throughout the test period by feeding standard feedstuff and drinking water. The cultured cells were subcutaneously inoculated into the dorsal region of the nude mice at a concentration of 3×10⁶ cells/0.1 mL/mouse. Nude mice which had the tumor volumes (minor axis × [major axis]² × 0.5) ranging from 123.3 mm³ to 274.4 mm³ were assigned by using SAS to minimize the fluctuation in the tumor volume between the groups and within the group.

### 2) Drug administration and measurement

The first day of administration was designated as Day 0, and observation was made until Day 59. The respective groups (n = 5) were treated as follows. FK330 was orally gavaged in an amount of 5 mL/kg, and paclitaxel was administered intravenously through the tail vein in an amount of 10 mL/kg. Paclitaxel was continuously administered from Day 0 to Day 5, and then was withdrawn for 21 days. This was defined as one cycle, and administration was carried out up to two cycles.

Control group: Untreated
Paclitaxel single administration group: Paclitaxel 10 mg/kg/day (continuously administered for six days from Day 0 in Cycle 1, and continuously administered for 6 days from Day 27 in Cycle 2)

Combined use group:
FK330 30 mg/kg bid (60 mg/kg/day) + paclitaxel 10 mg/kg/day (continuously administered for 6 days from Day 0 in Cycle 1, and continuously administered for 6 days from Day 27 in Cycle 2)
FK330 60 mg/kg bid (120 mg/kg/day) + paclitaxel 10 mg/kg/day (continuously administered for 6 days from Day 0 in Cycle 1, and continuously administered for 6 days from Day 27 in Cycle 2)
FK330 100 mg/kg bid (200 mg/kg/day) + paclitaxel 10 mg/kg/day (continuously administered for 6 days from Day 0 in Cycle 1, and continuously administered for 6 days from Day 27 in Cycle 2)
FK330 was orally gavaged (5 mL/kg) twice a day at an interval of 6 to 8 hours. Paclitaxel was administered by intravenous bolus injection (10 mL/kg) through the tail vein 2 to 4 hours after the administration of FK330.

Every 3 to 4 days, the body weight and the tumor diameters, determined by using a digital caliper, were measured. The tumor volume was calculated from the calculation formula for ellipsoid volume of (minor axis × [major axis]² × 0.52).

### 3) Results

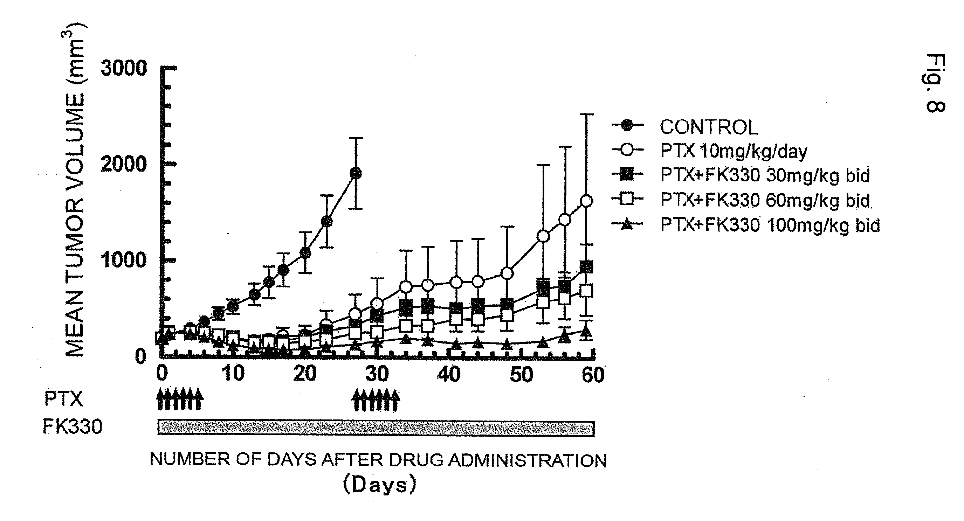

The results of investigating the dose-dependency of FK330 in the enhancing effect on anti-tumor effect of paclitaxel are shown in FIG 8. The results are expressed as the mean value (Mean) ± standard error (SEM) (n = 4 to 5). The tumor volume at the end of the study (Day 59) was 1640.1 ± 902.8 mm³ in the paclitaxel-only group; 955.9 ± 231.0 mm³ in the FK330 30 mg/kg bid combined use group; 706.2 ± 256.1 mm³ in the FK330 60 mg/kg bid combined use group; and 297.9 ± 101.4 mm³ in the FK330 100 mg/kg bid combined use group, and FK330 enhanced the anti-tumor effect of paclitaxel in a dose-dependent manner (tumor volume reduction ratio compared with the paclitaxel-only group: 41.7% in FK330 30 mg/kg bid; 56.9% in FK330 60 mg/kg bid, and 81.8% in FK330 100 mg/kg bid).

### 4) Conclusions

Combined use of FK330 enhanced the anti-tumor effect of paclitaxel in a dose-dependent manner.

### Example 4

Preparation of the test substances and conditioning of the cells were carried out in the same manner as in Example 2.

### 1) Animals

Five-week old male nude mice (CAnN Cg-Foxn1nu/CrlCrlj(nu/nu)) were purchased from Charles River Laboratories Japan, Inc. (Japan, Kanagawa). The animals were raised under specific pathogen-free (SPF) conditions throughout the test period by feeding standard feedstuff and drinking water. The cultured cells were subcutaneously inoculated into the dorsal region of the nude mice at a concentration of 3×10⁶ cells/0.1 mL/mouse. Nude mice which had the tumor volumes (minor axis × [major axis]² × 0.5) ranging from 142.1 mm³ to 323.7 mm³ were assigned by using SAS to minimize the fluctuation in the tumor volume between the groups and within the group.

### 2) Drug administration and measurement

The first day of administration was designated as Day 0, and observation was made until Day 55. The respective groups (n = 5 or 6) were treated as follows. FK330 was orally gavaged in an amount of 5 mL/kg, and paclitaxel was administered intravenously through the tail vein in an amount of 10 mL/kg. Paclitaxel was continuously administered from Day 0 to Day 5, was subsequently withdrawn for 21 days, and then was continuously administered for 5 days from Day 27 to Day 31.

Control group: Untreated
Paclitaxel single administration group: Paclitaxel 10 mg/kg/day (from Day 0 to Day 5, and from Day 27 to Day 31)

Combined use group:
Concomitant administration, FK330 100 mg/kg bid (200 mg/kg/day) + paclitaxel 10 mg/kg/day (from Day 0 to Day 5, and from Day 27 to Day 31)
Sequential administration, FK330 100 mg/kg bid (200 mg/kg/day) + paclitaxel 10 mg/kg/day (from Day 0 to Day 5, and from Day 27 to Day 31)
Continuous administration, FK330 100 mg/kg bid (200 mg/kg/day) + paclitaxel 10 mg/kg/day (from Day 0 to Day 5, and from Day 27 to Day 31)
FK330 was orally gavaged (5 mL/kg) twice a day at an interval of 6 to 8 hours.
In the Concomitant administration, FK330 was administered on the days of administration of paclitaxel (from Day 0 to Day 5, and from Day 27 to Day 31); and in the continuous administration, FK330 was administered on the withdrawal days after the days of administration of paclitaxel (from Day 6 to Day 26, and from Day 32 to Day 55), while the continuous administration was carried out for all days from the day of experiment initiation to the day of experiment completion. Paclitaxel was administered by intravenous bolus injection (10 mL/kg) through the tail vein 2 to 4 hours after the administration of FK330.

Every 3 to 4 days, the body weight and the tumor diameters, determined by using a digital caliper, were measured. The tumor volume was calculated from the calculation formula for ellipsoid volume of (minor axis × [major axis]² × 0.52).

### 3) Results

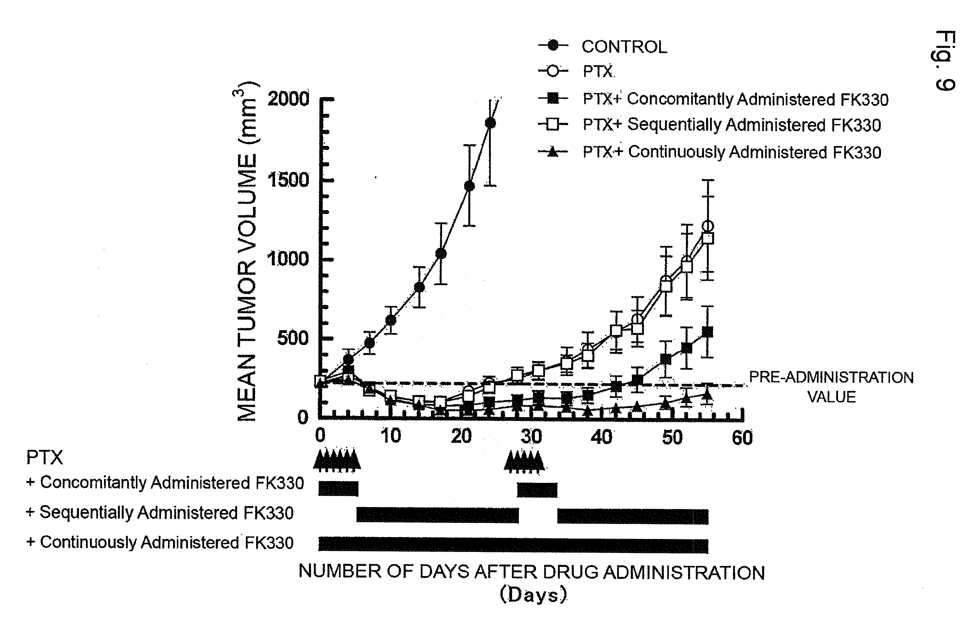

In human non-small cell lung cancer (Calu-6) tumor-bearing mice, the results of investigating the timing of administration of FK3 3 0 in the combined use with paclitaxel are shown in FIG 9. The results are expressed as the mean value (Mean) ± standard error (SEM) (n = 5 or 6).

During the test period, a value less than or equal to the palpation limit was defined as complete remission (CR), and the measurement value was designated as 0. The tumor volume at the end of the study (Day 55) was 1229.2 ± 289.6 mm³ in the paclitaxel-only group; 559.8 ± 161.8 mm³ in the concomitant administration of paclitaxel and FK330; 1150.9 ± 264.9 mm³ in the sequential administration of FK330; and 168.5 ± 66.0 mm³ in the continuous administration of FK330, and the tumor volume reduction ratio compared with the paclitaxel-only group was 54.5%, 6.4%, and 86.3%, respectively.

### 4) Conclusions

In regard to the timing of administration of FK3 3 0, it was suggested that the combined use effect was the highest when FK330 was administered for consecutive days during the cycle period from the day of initiation of administration of a taxane-family drug.

### Example 5

Preparation of the test substances was carried out in the same manner as in Example 2.

### 1) Cells

Human gastric cancer-derived NCI-N87 (CRL-5822) was obtained from American Type Culture Collection (VA, USA), MKN-28 (JCRB0253) was obtained from Health Science Research Resources Bank (HSRRB), and AZ-521 (RCB2087) was obtained from Riken BRC. NCI-N87 and MKN-28 cells were cultured under the conditions of 37°C and 5% CO₂ by using RPMI1640 medium containing 10% heat-inactivated fetal bovine serum (FBS), and AZ-521 cells were cultured under the conditions of 37°C and 5% CO₂ by using DMEM medium containing 10% heat-inactivated FBS. The cells were harvested with trypsin and were suspended in PBS at 6×10⁷ cells/mL, and the suspension was mixed with an equal amount of Matrigel (trade name) Basement Membrane Matrix (manufactured by Becton Dickinson Co., Bedford, MA, USA).

### 2) Animals

Five-week old male nude mice (CAnN Cg-Foxn1nu/CrlCrlj(nu/nu)) were purchased from Charles River Laboratories Japan, Inc. (Japan, Kanagawa). The animals were raised under specific pathogen-free (SPF) conditions throughout the test period by feeding standard feedstuff and drinking water. The cultured cells were subcutaneously inoculated into the dorsal region of the nude mice at a concentration of 3×10⁶ cells/0.1 mL/mouse. Nude mice which had the tumor volumes (minor axis × [major axis]² × 0.5) ranging from 386.6 mm³ to 766.6 mm³ in the case of AZ-521 tumor-bearing mice; the tumor volume ranging from 186.0 mm³ to 319.2 mm³ in the case of MKN-28 tumor-bearing mice; and the tumor volume ranging from 192.1 mm³ to 364.2 mm³ in the case of NCI-N87 tumor-bearing mice, were assigned by using SAS to minimize the fluctuation in the tumor volume between the groups and within the group.

### 3) Drug administration and measurement

The first day of administration was designated as Day 0, and observation was made until Day 46 in the AZ-521 tumor-bearing mice, until Day 84 in the MKN-28 tumor-bearing mice, and until Day 56 in the NCI-N87 tumor-bearing mice. The respective groups (n = 5 or 6) were treated as follows. FK330 was orally agavaged in an amount of 5 mL/kg, and paclitaxel was administered intravenously through the tail vein in an amount of 10 mL/kg. Paclitaxel was administered under the following multicycle treatment conditions.

Control group: Untreated
FK330 single administration group: FK330 100 mg/kg bid (200 mg/kg day, continuously administered from the initiation of experiment to the completion of experiment)
Paclitaxel single administration group: Paclitaxel 10 mg/kg day

AZ-521 tumor-bearing mice:
Paclitaxel was continuously administered for 6 days from Day 0 to Day 5, subsequently a drug withdrawal period of 22 days was taken (Cycle 1), and paclitaxel was continuously administered for 5 days from Day 28 to Day 32 (Cycle 2).

MKN-28 tumor-bearing mice:
Paclitaxel was continuously administered for 6 days from Day 0 to Day 5, subsequently a drug withdrawal period of 22 days was taken, subsequently paclitaxel was continuously administered for 5 days from Day 28 to Day 32, and a drug withdrawal of 23 days was taken (Cycle 2). Furthermore, continuous administration was carried out for 5 days from Day 56 to Day 60 (Cycle 3).

NCI-N87 tumor-bearing mice:
Paclitaxel was continuously administered for 6 days from Day 0 to Day 5, subsequently a drug withdrawal period of 26 days was taken, and then paclitaxel was continuously administered for 5 days from Day 32 to Day 36 (Cycle 2).

Combined use group:
FK330 100 mg/kg bid (200 mg/kg day, continuously administered from the initiation of experiment to the completion of experiment) + paclitaxel 10 mg/kg/day
FK330 was orally gavaged (5 mL/kg) twice a day at an interval of 6 to 8 hours. Paclitaxel was administered by intravenous bolus injection (10 mL/kg) through the tail vein 2 to 4 hours after the administration of FK330.

Every 3 to 4 days, the body weight and the tumor diameters, determined by using a digital caliper, were measured. The tumor volume was calculated from the calculation formula for ellipsoid volume of (minor axis × [major axis]² × 0.52).

### 4) Statistical analysis

The results are expressed as the mean value (Mean) ± standard error (SEM). Using the actual measured values of the tumor volume at each measurement time point, an unpaired statistical test (Student's t-test) of the difference between two groups was carried out between a single administration group and a combined use group. A difference value of less than 5% was considered to be statistically significant. For the data processing, Graphpad Prism version 5.03 was used.

### 5) Results

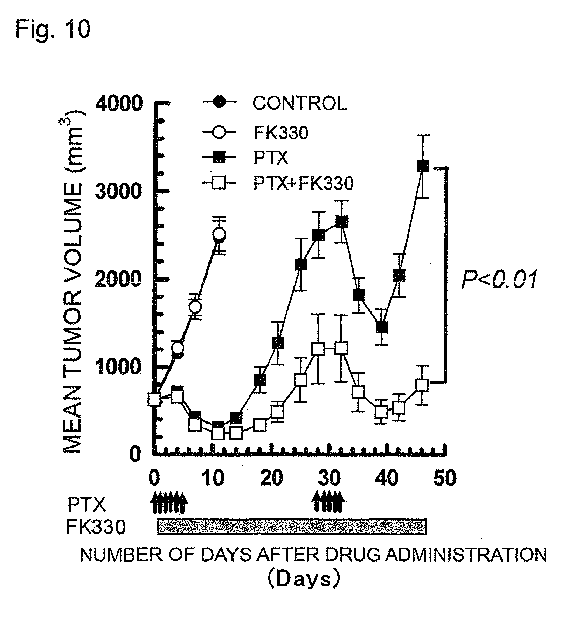

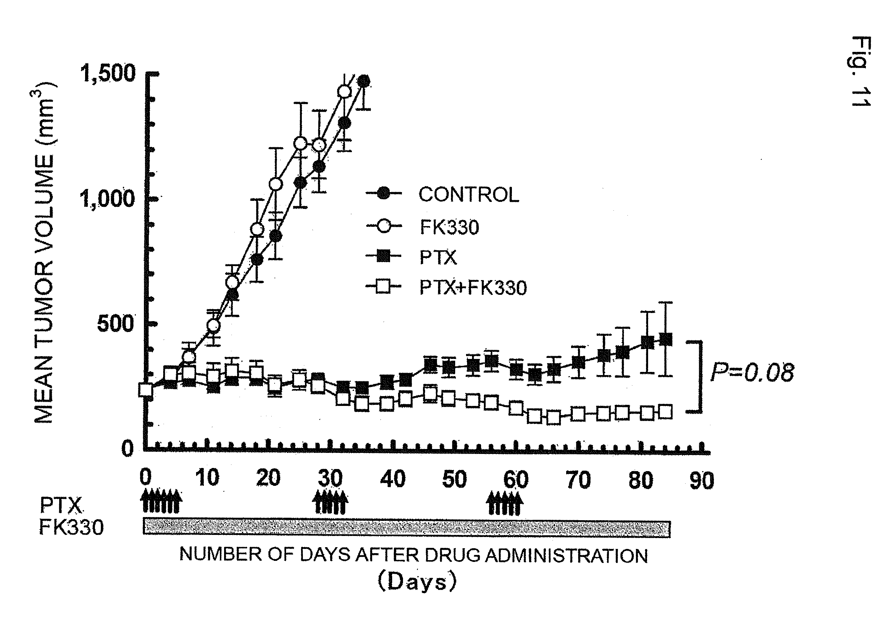

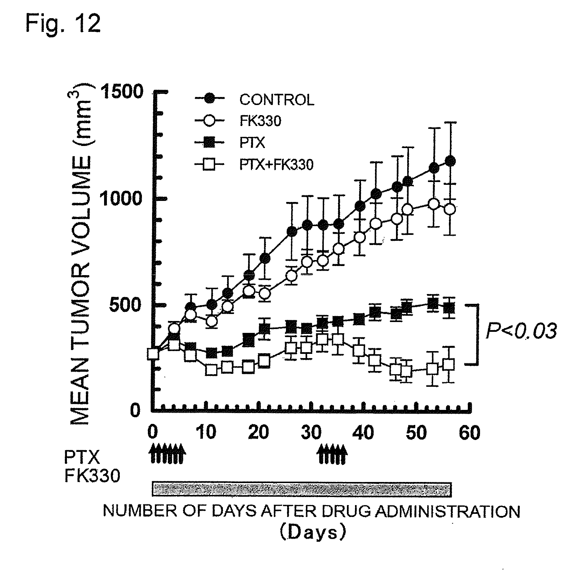

In human gastric cancer (AZ-521, MNK-28, and NCI-N87) tumor-bearing mice, the results of investigating the effect of FK330 of combined use with paclitaxel are shown in FIG. 10 to FIG 12. That is, the combined use effect of FK330 and paclitaxel in a AZ-521 tumor-bearing mice is shown in FIG. 10; the combined use effect in MKN-28 tumor-bearing mice is shown in FIG. 11; and the combined use effect in a NCI-N87 tumor-bearing mice is shown in FIG. 12. Similarly to the case of the human non-small cell lung cancer tumor-bearing mice, FK330 enhanced the anti-tumor effect of paclitaxel. In the AZ 521 tumor-bearing mice, the tumor volume of the paclitaxel-only group on the last day of experiment was 3285.4 ± 359.4 mm³, and the tumor volume of the combined used group of paclitaxel and FK330 was 795.0 ± 223.3 mm³. Thus, the tumor volume decreased by 75.8% (P < 0.01) compared with the paclitaxel-only group. Also in the MKN-28 and NCI-N87 tumor-bearing mice, FK330 enhanced the anti-tumor effect of paclitaxel (MKN-28 tumor-bearing mice: 454.1 ± 147.5 mm³ vs. 164.8 ± 18.5 mm³, decrease by 63.7%, P = 0.08, NCI-N87 tumor-bearing mice: 492.0 ± 48.5 mm³ vs. 223.9 ± 85.1 mm³, decrease by 54.5%, P < 0.03).

The results are expressed as the mean value (Mean) ± standard error (SEM) (n = 5 or 6). The P value represents the significant difference obtained as compared between a combined use group and a chemotherapeutic agent single administration group (Student's t-test).

### 6) Conclusions

Combined use of FK330 enhanced the anti-tumor effect of paclitaxel in a human gastric cancer tumor-bearing model, and particularly, an effect of remedying the attenuation of the anti-tumor effect of paclitaxel through multicycle treatment was recognized.

### Example 6

### 1) Test substances

The dose of FK330 is expressed in terms of the weight of a simple substance (non-hydrate). Paclitaxel was purchased from LC Laboratories, Inc. (Woburn, MA, USA), and was used as paclitaxel.

### 2) Preparation and administration of test substances

FK330 was suspended (20 mg/mL) in a 0.5% aqueous methyl cellulose solution (0.5% MC), and was orally administered. Paclitaxel was prepared into a stock solution at 10 mg/mL with an ethanol/chromophore solution (mixed at 1:1), and then the stock solution was diluted to 1 mg/mL with physiological saline for injection (Otsuka Pharmaceutical Group) immediately before administration and was intravenously administered through the tail vein.

### 3) Cells

Human breast cancer-derived MDA-MB-231 (HTB-26) was obtained from American Type Culture Collection (VA, USA). The cells were cultured under the conditions of 37°C and 5% CO₂ by using DMEM medium containing 10% heat-inactivated fetal bovine serum (FBS). The cells were harvested with trypsin and were suspended in PBS at 6×10⁷ cells/mL, and the suspension was mixed with an equal amount of Matrigel (trade name) Basement Membrane Matrix (manufactured by Becton Dickinson Co., Bedford, MA, USA).

### 4) Animals

Five-week old female nude mice (CAnN Cg-Foxn1nu/CrlCrlj(nu/nu)) were purchased from Charles River Laboratories Japan, Inc. (Japan, Kanagawa). The animals were raised under specific pathogen-free (SPF) conditions throughout the test period by feeding standard feedstuff and drinking water. The cultured cells were subcutaneously inoculated into the dorsal region of the nude mice at a concentration of 3×10⁶ cells/0.1 mL/mouse. Nude mice which had the tumor volumes (minor axis × [major axis]² × 0.5) ranging from 148.7 mm³ to 312.5 mm³ were assigned by using SAS to minimize the fluctuation in the tumor volume between the groups and within the group.

### 5) Drug administration and measurement

The first day of administration was designated as Day 1, and observation was made until Day 66. The respective groups (n = 6) were treated as follows. FK330 was orally gavaged in an amount of 5 mL/kg, and paclitaxel was administered intravenously through the tail vein in an amount of 10 mL/kg. Paclitaxel was continuously administered for 4 days from Day 1 to Day 4, and then was withdrawn for 25 days (Cycle 1). In the administration of PTX in Cycle 2, the drug was continuously administered for 4 days from Day 30.

Control group: untreated
FK330 single administration group: FK330 100 mg/kg bid (200 mg/kg day, continuously administered from the initiation of experiment to the completion of experiment)
Paclitaxel single administration group: Paclitaxel 10 mg/kg/day (continuously administered for 4 days in each cycle)

Combined use group:
FK330 100 mg/kg/kg bid (200 mg/kg day, continuously administered from the initiation of experiment to completion of experiment) + paclitaxel 10 mg/kg day (continuously administered for 4 days in each cycle)
FK330 was orally gavaged (5 mL/kg) twice a day for 6 to 8 hours. Paclitaxel was administered by intravenous bolus injection (10 mL/kg) through the tail vein 1 to 4 hours after the administration of FK330.

The body weight and the tumor diameter, determined by using a digital caliper, were measured twice a week. The tumor volume was calculated from the calculation formula for ellipsoid volume of (minor axis × [major axis]² × 0.52).

### (6) Statistical analysis

The results are expressed as the mean value (Mean) ± standard error (SEM). During the test period, a value less than or equal to the palpation limit was defined as complete remission (CR), and the measurement value was designated as 0. Using the actual measured values of the tumor volume at each measurement time points, an unpaired statistical test (Student's t-test) of the difference between two groups was carried out between a single administration group and a combined use group. A difference value of less than 5% was considered to be statistically significant. For the data processing, Graphpad Prism version 5.03 was used.

### 7) Results

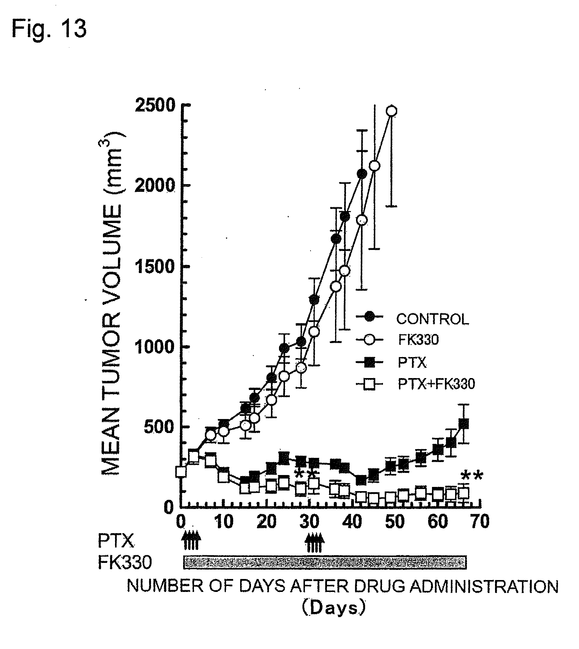

The results of investigating the combined use effect of FK330 with paclitaxel in human breast cancer tumor-bearing mice are shown in FIG. 13. In human breast cancer (MDA-MB-231) tumor-bearing mice, FK330 clearly enhanced the anti-tumor effect of paclitaxel, and the tumor volume reduction ratio compared with the paclitaxel-only group on Day 28 was 59.6% (P < 0.01), and the tumor volume reduction ratio compared with the paclitaxel-only group on Day 66 was 82.8% (P < 0.01).

The results are expressed as the mean value (Mean) ± standard error (SEM) (n = 6). **: P < 0.01 represents the significant difference obtained as compared between the combined use group and the paclitaxel single administration group (Student's t-test).

### 8) Conclusions

Combined use of FK330 enhanced the anti-tumor effect of paclitaxel under its multicycle-administration in human breast cancer (MDA-MB-231) tumor-bearing mice, and very clearly suppressed regrowth of tumor.

### Example 7

### 1) Test substances

S-nitroso-N-acetyl-D,L-penicillamine (SNAP), which is a NO donor, was purchased from Cayman Chemical Co. (Ann Arbor, MI, USA) and used. A tubulin polymerization analysis kit was purchased from Cytoskeleton, Inc. (Denver, CO, USA) and used. For paclitaxel, a paclitaxel included in the analysis kit was used as paclitaxel. Docetaxel was purchased from Sigma-Aldrich Co. (St. Louis, MO, USA) and was used as docetaxel.

### 2) Test method

A fluorescence-based assay of tubulin polymerization reaction was carried out according to the manufacturer's instruction of the kit. That is, purified pig brain-derived tubulin (final concentration: 2 mg/mL) and SNAP (final concentration: 200 or 500 µmol/L) were incubated at room temperature for 15 minutes or longer in the buffer solution included in the kit. Subsequently, paclitaxel (final concentration: 3 µmol/L) or docetaxel (final concentration: 1 µmol/L), and GPT (final concentration: 1 mmol/L) were added to this buffer mixture, and then was incubated for 60 minutes at 37°C. During this incubation, the fluorescence intensity (measurement wavelength: excitation 350 nm/fluorescence 435 nm) was measured every one minute by using a microplate reader (Spectramax, Molecular Devices, LLC, Sunnyvale, CA, USA). The value of fluorescence intensity was expressed as a percentage relative to the SNAP and taxane-untreated group by using the value obtained 60 minutes after the initiation of the reaction, which corresponded to the equilibrium steady-state phase, and the average value for three experiments was taken as the value.

### 3) Statistical analysis

The results are expressed as the mean value (Mean) ± standard error (SEM) of five independent experiments. A comparison between a paclitaxel- or docetaxel-treated group and an untreated group was carried out by an unpaired statistical test (Student's t-test) of the difference between two groups. A comparison between a SNAP-untreated group (control group) and SNAP-treated groups (200 µmol/L and 500 µmol/L) in the presence or absence of the taxane-family drugs was carried out by Dunnett's multiple comparison test. A difference value of less than 5% was considered to be statistically significant. For the data processing, Graphpad Prism version 5.03 was used.

### 4) Results

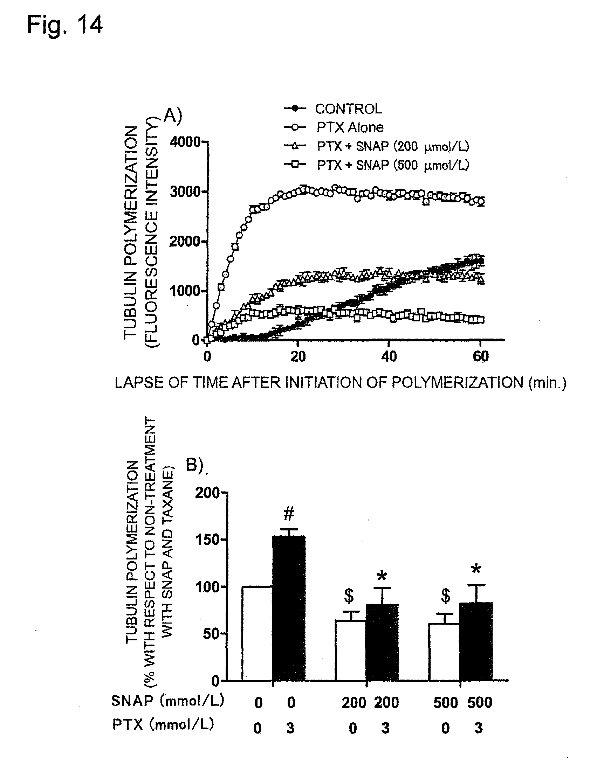

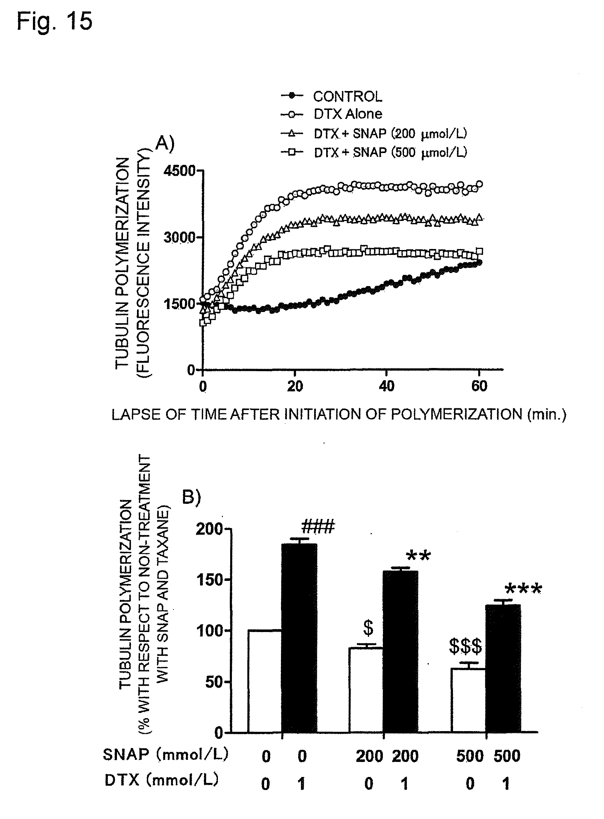

A representative example of the suppressive action of SNAP on the paclitaxel-induced tubulin polymerization action, and the summary values for 60 minutes after the initiation of tubulin polymerization, are shown in FIG 14(A) and FIG. 14(B). A representative example of the suppressive action of SNAP on the docetaxel-induced tubulin polymerization action, and the summary values for 60 minutes after the initiation of tubulin polymerization, are shown in FIG. 15(A) and FIG. 15(B). Paclitaxel or docetaxel significantly promoted tubulin polymerization at 152.7% (P < 0.05) and 184.1% (P < 0.001), respectively, as compared with the respective taxane-untreated groups. SNAP as a NO donor significantly suppressed the tubulin polymerization reaction promoted by paclitaxel and docetaxel. Furthermore, SNAP also significantly suppressed the spontaneous tubulin polymerization reaction.

The results are expressed as the mean value (Mean) ± standard error (SEM) (n = 5). #: P < 0.05 and ###: P < 0.001 represent the significant differences obtained as compared between the paclitaxel- or docetaxel-treated group and the corresponding taxane drug-untreated group (Student's t-test). $: P < 0.05 and $$$: P < 0.001 represent the significant differences of the SNAP-treated group as compared to the SNAP-untreated group (control group) in the absence of taxanes (Dunnett's multiple comparison test). *: P < 0.05, **; P < 0.01, and ***: P < 0.001 represent the significant differences of the SNAP-treated group as compared to the SNAP-untreated group (control group) in the presence of taxanes (Dunnett's multiple comparison test).

### 5) Conclusions

The NO donor directly suppressed the tubulin polymerization reaction promoted by paclitaxel and docetaxel.

### Example 8

### 1) Test substances

The dose of FK330 is expressed in terms of the weight of a simple substance (non-hydrate). A paclitaxel injectable preparation (paclitaxel injection liquid "Sawai") was purchased from Sawai Pharmaceutical Co., Ltd., and was used as paclitaxel. Rat anti-mouse F4/80 antigen monoclonal antibody, rabbit anti-iNOS polyclonal antibody, and rabbit anti-nitrotyrosine antibody were respectively purchased from AbD Serotec (Oxford, UK), Santa Cruz Biotechnology, Inc. (Santa Cruz, CA, US), and Millipore Corp. (Billerica, MA, USA) and used.

### 2) Preparation and administration of test substances

FK330 was suspended (20 mg/mL) in a 0.5% aqueous methyl cellulose solution (0.5% MC) and orally administered. Paclitaxel was prepared with physiological saline for injection (Otsuka Pharmaceutical Group) to a concentration of 1 mg/mL at the time of use.

### 3) Cells

Human lung cancer-derived Calu-6 (HTB-56) was obtained from American Type Culture Collection (VA, USA). The cells were cultured under the conditions of 37°C and 5% CO₂ by using RPMI1640 medium containing 10% heat-inactivated fetal bovine serum (FBS). The cells were harvested with trypsin and were suspended in PBS at 6×10⁷ cells/mL, and the suspension was mixed with an equal amount of Matrigel (trade name) Basement Membrane Matrix (manufactured by Becton Dickinson Co., Bedford, MA, USA).

### 4) Animals

Five-week old male nude mice (CAnN Cg-Foxn1nu/CrlCrlj(nu/nu)) were purchased from Charles River Laboratories Japan, Inc. (Japan, Kanagawa). The animals were raised under specific pathogen-free (SPF) conditions throughout the test period by feeding standard feedstuff and drinking water. The cultured cells were subcutaneously inoculated into the dorsal region of the nude mice at a concentration of 3×10⁶ cells/0.1 mL/mouse. Nude mice which had the tumor volumes (minor axis × [major axis]² × 0.5) ranging from 136.8 mm³ to 248.5 mm³ were assigned by using SAS to minimize the fluctuation in the tumor volume between the groups and within the group.

### 5) Drug administration

Drug administration was initiated from the first day of administration (Day 1). The respective groups (n = 5) were treated as follows. FK330 was orally gavaged in an amount of 5 mL/kg from Day 1 to Day 6, and paclitaxel was intravenously administered through the tail vein in an amount of 10 mL/kg from Day 1 to Day 5.

Control group: untreated
FK330 single administration group: FK330 100 mg/kg bid (200 mg/kg/day, from Day 1 to Day 6)
Paclitaxel single administration group: Paclitaxel 10 mg/kg/day (from Day 1 to Day 5)
Combined use group: FK330 100 mg/kg/kg bid (200 mg/kg/day, from Day 1 to Day 6) + paclitaxel 10 mg/kg/day (from Day 1 to Day 5)
FK330 was orally gavaged (5 mL/kg) twice a day at an interval of 6 to 8 hours. Paclitaxel was administered by intravenous bolus injection (10 mL/kg) through the tail vein 1 to 4 hours after the administration of FK330.

### 6) Tissue fixation and preparation of frozen sections

On Day 6, the tumor tissues were harvested from the mice. Immersion fixation was carried out at 4°C for 12 hours with a 4% para-formaldehyde-phosphate buffer solution (Wako Pure Chemical Industries, Ltd.). After the tissue fixation, according to the conventional method, sucrose replacement was carried out using 10%, 15% and 20% sucrose/phosphate buffer solutions in a stepwise manner at 4°C. After completion of the replacement, the cancer tissues were embedded in O.C.T. Compound (Toronto Research Chemicals, Inc., Toronto, Canada), and frozen sections were prepared with dry ice/acetone and stored at -80°C.

### 7) Immunochemical staining and image analysis

Frozen sections at 5 µm thickness were prepared by a cryostat (Sakura Finetech Japan Co., Ltd.) and were air-dried. The dried frozen sections were washed with a 4% para-formaldehyde-phosphate buffer solution, and then in order to inactivate the endogenous peroxidase activity, the sections were treated for 30 minutes with 0.3% hydrogen peroxide/methanol. Mouse macrophage, iNOS and nitrotyrosine staining was carried out by the following procedure. In regard to the iNOS and nitrotyrosine staining, antigen retrieval was carried out by microwave irradiation (3 minutes/500 W) before the immunochemical staining.

Mouse macrophage staining:
Primary antibody treatment: Incubated with rat anti-mouse F4/80 antigen monoclonal antibody (1:200 dilution) for 60 minutes at room temperature.
Secondary antibody treatment: Incubated with biotinylated anti-rat IgG antibody (1:100 dilution, Vector Laboratories, Inc.) for 30 minutes at room temperature.
Detection system: The sections were incubated by using a Vectastain ABC kit (Vector Laboratories, Inc.) for 30 minutes at room temperature.
Chromogenic method: The sections were treated by using a 3,3'-diaminobenzidine tetrahydrochloride (DAB, Dako, Inc.) reagent at room temperature for 1 minute.

iNOS staining:
Primary antibody treatment: Incubated with rabbit iNOS polyclonal antibody (1:500 dilution) for one day and night at 4°C.
Detection system: The sections were incubated by using EnVision (Dako, Inc.) for 30 minutes at room temperature.
Chromogenic method: The sections were treated by using a DAB reagent for 1 minute at room temperature.

Nitrotyrosine staining:
Primary antibody treatment: Incubated with rabbit anti-nitrotyrosine antibody (1:500 dilution) for one day and night at 4°C.
Detection system: The sections were incubated by using EnVision (Dako, Inc.) for 30 minutes at room temperature.
Chromogenic method: The sections were treated by using a DAB reagent for 1 minute at room temperature.

The stained images (F4/80 stained images and iNOS images were magnified 200 times, and the nitrotyrosine stained images were magnified 400 times) were input into a computer through a microscope, and were analyzed by means of an image analysis system (Mitani Corp.) mounted with WinROOF (version 5.7). The values are expressed as the ratio (%) of the positive area per total area. Five fields of vision were analyzed in each section, and the mean values were taken as the value.

### 8) Statistical analysis

The results are expressed as the mean value (Mean) ± standard error (SEM). For the respective measured values of the iNOS protein expression, macrophage (F4/80) infiltration, and nitrotyrosine (major end-product of NO) in the tumor tissue, in a comparison of the control group and the paclitaxel-alone group, the FK330-only group or the paclitaxel and FK330 combined use group, and a comparison of the paclitaxel-only group and the paclitaxel and FK330 combined use group, an unpaired statistical test (Student's t-test) of the difference between two groups was carried out. A difference value of less than 5% was considered to be statistically significant. For the data processing, Graphpad Prism version 5.03 was used.

### 9) Results

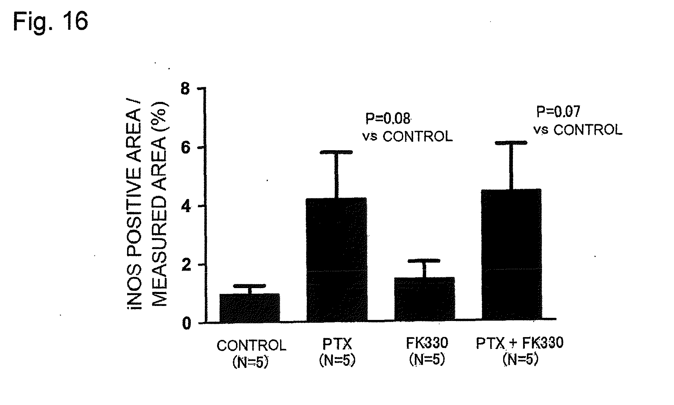

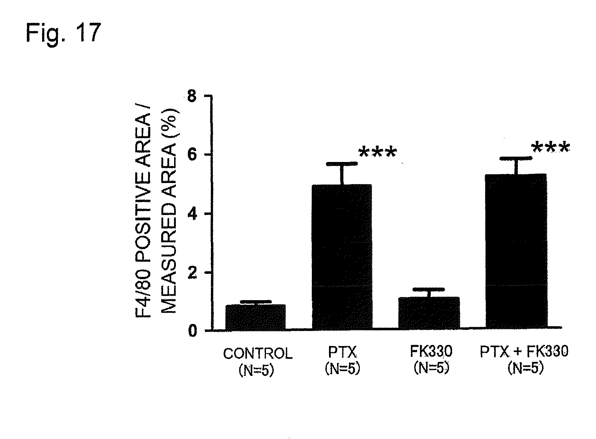

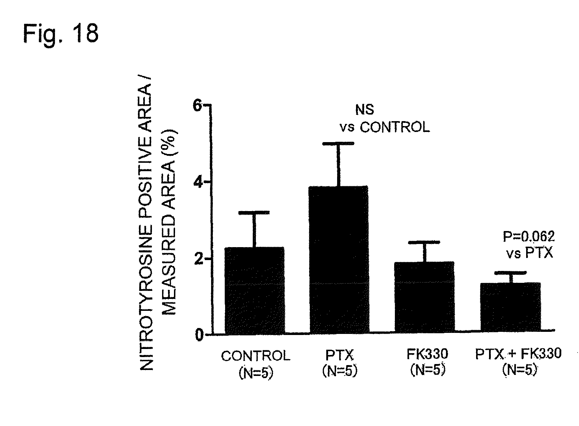

In human lung cancer cell line Calu-6 tumor-bearing mice, the effects on the iNOS protein expression, macrophage (F4/80) infiltration, and nitrotyrosine (major end-product of NO) production in the tumor tissue when treated with paclitaxel alone and the combination with FK330 are shown in FIG. 16 to FIG 18. In the tumor tissue of the control group, iNOS positive cells and macrophages were not recognized in most cases, and the level of nitrotyrosine (major end-product NO) was also low. On the other hand, when paclitaxel was administered, infiltration of macrophages in the tumor tissue was clearly recognized. Also, iNOS protein expression in the tumor tissue was also very clearly accentuated, and as a result, the amount of nitrotyrosine in the tumor tissue was also increased. Combined use of FK330, which is an iNOS inhibitor, did not affect the macrophage infiltration and iNOS protein expression in the tumor, but suppressed nitrotyrosine production.

The results are expressed as the mean value (Mean) ± standard error (SEM) (n = 5). ***: P < 0.001 represents the significant difference obtainable as compared with the control group (Student's t-test).

### 10) Conclusions

In human lung cancer (Calu-6) tumor-bearing mice, paclitaxel induced macrophage infiltration and iNOS protein expression in the tumor tissue, and consequently induced NO production in the tumor tissue. Combined use with FK330, which is an iNOS inhibitor, suppressed the iNOS-derived NO production induced by paclitaxel.

### Industrial Applicability

The composition for cancer treatment of the present invention comprising FK330 or a salt thereof as an active ingredient, which is characterized in being used in combined administration with a taxane-family anti-tumor agent, can obtain a superior cancer treating effect when used in combination with a taxane-family anti-tumor agent. Thus, the composition for cancer treatment is useful particularly in the treatment of various cancers for which it is known that existing taxane-family anti-tumor agents are being applied. Applicable cancers include solid tumors and lymphoma for which taxane-family anti-tumor agents are used, and according to an embodiment, examples of the cancer include breast cancer, uterine cancer, ovarian cancer, prostatic cancer, lung cancer, gastric (gastric gland) cancer, non-small cell lung cancer, pancreatic cancer, head and neck squamous cell cancer, esophageal cancer, urinary bladder cancer, melanoma, colon cancer, renal cell cancer, and non-Hodgkin's lymphoma.

## Claims

1. A composition for cancer treatment, comprising N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof as an active ingredient, which is used for combined administration in combination with one or more anti-tumor agents selected from taxane-family anti-tumor agents.

2. The composition according to claim 1, which is used for combined administration in combination with paclitaxel or docetaxel.

3. The composition according to claim 2, which is used for combined administration in combination with paclitaxel.

4. The composition according to claim 2, which is used for combined administration in combination with docetaxel.

5. The composition according to any one of claims 1 to 4, which is a composition for oral administration comprising N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof in an amount of 60 mg/day to 1200 mg/day.

6. The composition according to claim 5, which is a composition for oral administration comprising N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof in an amount of 60 mg/day to 200 mg/day.

7. The composition according to any one of claims 1 to 6, which is used for combined administration in further combination with one or more anti-tumor agents selected from platinum-family anti-tumor agents, anthracycline-family anti-tumor agents, cyclophosphamide, fluorouracil, trastuzumab, and capecitabine.

8. The composition for cancer treatment according to any one of claims 1 to 6, which is administered together with one or more anti-tumor agents selected from taxane-family anti-tumor agents simultaneously, separately, continuously or with an interval.

9. A composition for cancer treatment for treating a subject undergoing therapy with one or more anti-tumor agents selected from taxane-family anti-tumor agents, wherein the composition comprises N²-[(2E)-3-(4-chlorophenyl)-2-propenoyl]-N-(2-oxo-2-{4-{[6-(trifluoromethyl)pyrimidin-4-yl]oxy}piperidin-1-yl)ethyl]-3-pyridin-2-yl-L-alaninamide or a salt thereof as an active ingredient.

10. The composition for cancer treatment according to any one of claims 1 to 9, which is for the treatment of cancer in which taxane-family anti-tumor agents are used.

11. The composition for cancer treatment according to claim 10, wherein the cancer for which the taxane-family anti-tumor agents are used is non-small cell lung cancer.

12. The composition for cancer treatment according to claim 10, wherein the cancer for which the taxane-family anti-tumor agents are used is breast cancer.

13. Use of N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof, for the manufacture of a composition for cancer treatment, which is used for combined administration in combination with one or more anti-tumor agents selected from taxane-family anti-tumor agents.

14. N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof, for use of cancer treatment, which is used for combined administration in combination with one or more anti-tumor agents selected from taxane-family anti-tumor agents.

15. A method for treating cancer, comprising combining one or more anti-tumor agents selected from taxane-family anti-tumor agents in a therapeutically effective amount that is used for combination therapy, and N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof in a therapeutically effective amount that is used for combination therapy; and administering the combination to a subject.

16. A method for treating cancer, comprising administering one or more anti-tumor agents selected from taxane-family anti-tumor agents in a therapeutically effective amount that is used for combination therapy, and N²-[(2E)-3-{4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof in a therapeutically effective amount that is used for combination therapy, to a subject simultaneously, separately, continuously, or with an interval.

17. An anti-tumor effect enhancer for one or more anti-tumor agents selected from taxane-family anti-tumor agents, which comprises N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof as an active ingredient.

18. An agent for suppressing or preventing resistance to one or more anti-tumor agents selected from taxane-family anti-tumor agents, which comprises N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof as an active ingredient.

19. A sensitivity enhancer for one or more anti-tumor agents selected from taxane-family anti-tumor agents, which comprises N²-[(2E)-3-(4-chlorophenyl)-2-propenoyl]-N-[2-oxo-2-(4-{[6-(trifluoromethyl)pyrimidin-4-yl]oxy}piperidin-1-yl)ethyl]-3-pyridin-2-yl-L-alaninamide or a salt thereof as an active ingredient.
